(19) Europäisches Patentamt
European Patent Office
Office européen des brevets

(11) EP 4 450 486 A1

(12) EUROPEAN PATENT APPLICATION
published in accordance with Art. 153(4) EPC

(43) Date of publication:
**23.10.2024 Bulletin 2024/43**

(21) Application number: **21968295.2**

(22) Date of filing: **22.12.2021**

(51) International Patent Classification (IPC):
***C07C 311/06*** (2006.01) ***C07C 311/14*** (2006.01)
***C07C 311/17*** (2006.01) ***C07C 321/28*** (2006.01)
***C07C 323/22*** (2006.01) ***C07C 323/66*** (2006.01)
***C07C 25/18*** (2006.01) ***C07D 319/20*** (2006.01)
***G03F 7/004*** (2006.01)

(52) Cooperative Patent Classification (CPC):
**C07C 25/18; C07C 311/06; C07C 311/14; C07C 311/17; C07C 321/28; C07C 323/22; C07C 323/66; C07D 319/20; G03F 7/004**

(86) International application number:
**PCT/KR2021/019567**

(87) International publication number:
**WO 2023/113086 (22.06.2023 Gazette 2023/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.12.2021 KR 20210181042**

(71) Applicant: **Dongjin Semichem Co., Ltd.**
**Incheon 22824 (KR)**

(72) Inventors:
- **KIM, Jaehyun**
  **Hwaseong-si, Gyeonggi-do 18635 (KR)**
- **HUR, Myoung Hyun**
  **Hwaseong-si, Gyeonggi-do 18635 (KR)**
- **KIM, Jeong Sik**
  **Hwaseong-si, Gyeonggi-do 18635 (KR)**
- **YOO, Min Ja**
  **Hwaseong-si, Gyeonggi-do 18635 (KR)**
- **LEE, Hyung Kun**
  **Hwaseong-si, Gyeonggi-do 18635 (KR)**

(74) Representative: **Grünecker Patent- und Rechtsanwälte**
**PartG mbB**
**Leopoldstraße 4**
**80802 München (DE)**

(54) **SALT COMPOUND, AND QUENCHER AND PHOTORESIST COMPOSITION COMPRISING SAME**

(57) The present disclosure relates to: a salt compound comprising an anion having a specific structure represented by Chemical Formula 1, and a cation represented by Chemical Formula 2; a quencher including the salt compound; and a photoresist composition including the salt compound.

EP 4 450 486 A1

## Description

### [TECHNICAL FIELD]

The present disclosures relates to a salt compound, a quencher and a photoresist composition comprising the same. More specifically, the present disclosure relates to a novel photosensitive salt compound used in chemically amplified resist materials, a quencher and a photoresist composition comprising the same.

### [BACKGROUND ART]

Lithography technology is a technology in which a resist material made of a photosensitive material is applied onto a substrate to form a resist film, and a patterned mask is exposed and developed on this resist film using a selected light source, so that a mask pattern is transferred to a resist film to form a pattern.

Semiconductor devices manufactured using a lithography technology use a short-wavelength light source so as to create finer patterns of semiconductor devices. For example, in the past, near-ultraviolet rays (g-line (436nm), i-line (365nm)) were used, but currently, deep ultraviolet (KrF (248 nm), ArF (193 nm) excimer lasers are used. ArF immersion and multi-patterning methods using the same are widely used in the mass production of semiconductor devices. Recently, a lithography technology using extreme ultraviolet (EUV (13.5 nm)) wavelengths has also begun to be introduced into mass production processes.

Resist materials are required to have characteristics such as sensitivity to an exposure light source and resolving power to reproduce fine mask patterns. As the resist components that can exhibit these characteristics, a chemically-amplified photoresist(CAR) composition comprising: a photoacid generator that generates acid by an exposure process, a polymer resin that changes solubility in an alkaline developer solution due to the action of the generated acid, a quencher that can adjust the acid diffusion distance, and a solvent that can dissolve respective constituent components is used.

In the field of conventional chemically-amplified photoresist compositions, nitrogen-containing organic substances such as amines and amides, which can adjust the diffusion distance of the acid generated from the photoacid generator, have been used to improve resist performance.

However, recently, instead of nitrogen-containing organic compounds, photodegradable acid diffusion inhibitors that have a structure similar to that of photoacid generators and are decomposed by the exposure light source have been developed. Such photodegradable base acid diffusion inhibitors function as a quencher that suppresses acid diffusion in the non-exposed area, and photolyze and function as acids in the exposed area, thereby keeping the acid concentration in the exposed area relatively high, increasing the acid distribution contrast between the exposed area and the non-exposed area, and improving lithographic characteristics.

However, forming patterns of desired sizes from resist materials still has not reached an ideal degree. In this regard, there is still a need to develop a photosensitive quencher having a new structure to ensure high contrast during fine pattern formation.

### [DETAILED DESCRIPTION OF THE INVENTION]

### [Technical Problem]

It is an object of the present disclosure to provide a salt compound which makes it possible to obtain a photoresist composition, that has improved polarity and thus shortens the diffusion length of anions generated during exposure, ensures high contrast, improves the marginal resolving power, and further has low sensitivity, LER values and high process margins.

It is another object of the present disclosure to provide a quencher comprising the salt compound.

It is yet another object of the present disclosure to provide a photoresist composition comprising the salt compound.

### [Technical Solution]

In one aspect of the present disclosure, there is provided a salt compound comprising an anion represented by the following Chemical Formula 1, and a cation represented by the following Chemical Formula 2:

[Chemical Formula 1]

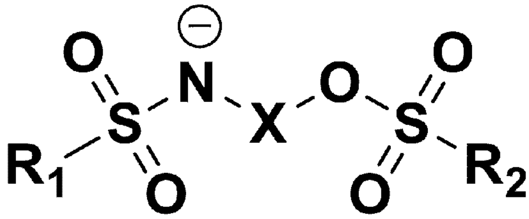

[Chemical Formula 2]

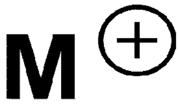

In another aspect of the present disclosure, there is provided a quencher comprising the salt compound.

In another aspect of the present disclosure, there is provided a photoresist composition comprising the salt compound.

In yet another aspect of the present disclosure, there is provided a method of forming a photoresist pattern using the photoresist composition.

Now, a salt compound, a quencher comprising the same, a photoresist composition comprising the same, and a method of forming a photoresist pattern according to specific embodiments of the disclosure will be described in more detail.

In the present disclosure, when a portion is referred to as "including" or "comprising" a certain component, it means that the portion can further include other components, without excluding the other components, unless otherwise stated.

In the present disclosure, the term "polymer" may be used interchangeably with the term "macromolecule" or "high molecule".

In the present disclosure, examples of substituents are explained below, but are not limited thereto.

In the present disclosure, the term "substituted" means that another atom or functional group is bonded in place of a hydrogen atom or carbon atom in a compound. The position to be substituted is not limited as long as it is a position where a hydrogen atom or a carbon atom is substituted, that is, a position where a substituent can be substituted. When two or more substituents are substituted, the two or more substituents may be the same as or different from each other.

In the present disclosure, the term "substituted or unsubstituted" means being unsubstituted or substituted with one or more substituent groups selected from the group consisting of deuterium; halogen; a cyano group; a nitro group; a hydroxy group; a carbonyl group; an ester group; an imide group; an amide group; an amino group; a carboxyl group; a sulfonic acid group; a sulfonamide group; a phosphine oxide group; an alkoxy group; an alkylcarbonyl group; an alkoxycarbonyl group; a sulfonyloxy group; an aryloxy group; an alkylthioxy group; an arylthioxy group; an alkylsulfoxy group; an arylsulfoxy group; a silyl group; and a boron group; or being unsubstituted or substituted with a substituent group to which two or more substituent groups of the above-exemplified substituent groups are linked. For example, "a substituent group in which two or more substituents are linked" may be a biphenyl group. Namely, a biphenyl group may be an aryl group, or it may be interpreted as a substituent group in which two phenyl groups are linked.

In the present disclosure, the notation

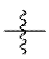 or 

means a bond linked to another substituent group.

In the present disclosure, examples of a halogen group include fluorine, chlorine, bromine, or iodine.

In the present disclosure, the carbon number of an imide group is not particularly limited, but is preferably 1 to 30. Specifically, the imide group may be a compound having the following structural formulas, but is not limited thereto.

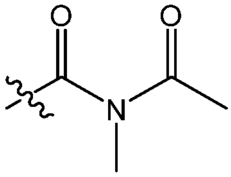 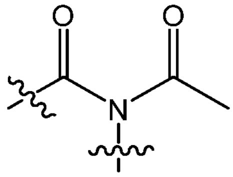 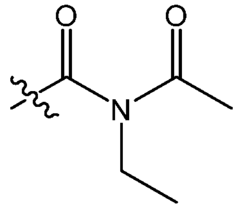

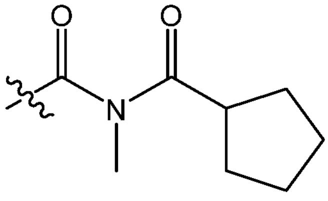 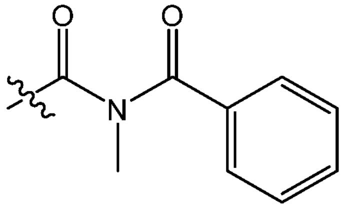

In the present disclosure, an amide group may have a structure in which nitrogen of the amide group may be substituted with hydrogen, a straight-chain, branched-chain, or cyclic alkyl group having 1 to 30 carbon atoms, or an aryl group having 6 to 30 carbon atoms. Specifically, the amide group may be a compound having the following structural formula, but is not limited thereto.

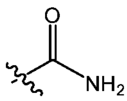 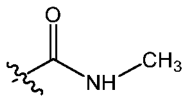 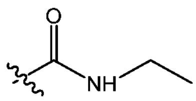 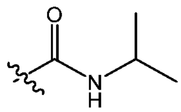

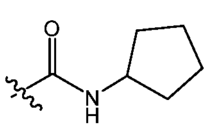 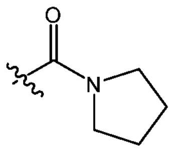 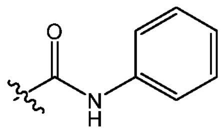 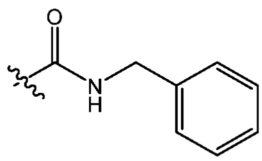

In the present disclosure, the carbon number of a carbonyl group is not particularly limited, but is preferably 1 to 30. Specifically, the carbonyl group may be a compound having the following structural formulas, but is not limited thereto.

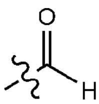 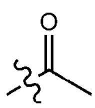 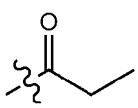 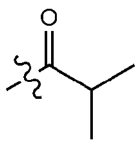

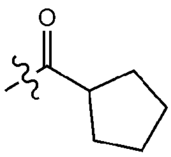 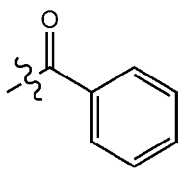 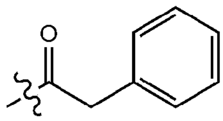

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a compound having the following structural formulas, but is not limited thereto.

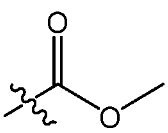 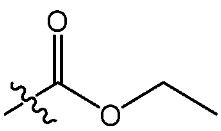 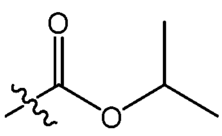 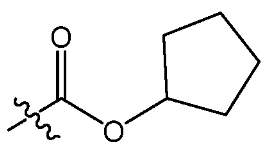

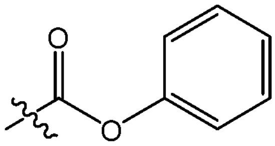 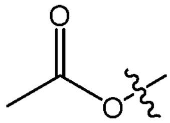 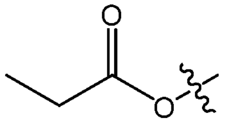 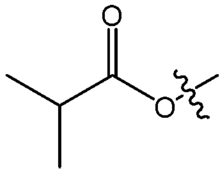

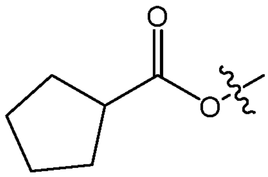 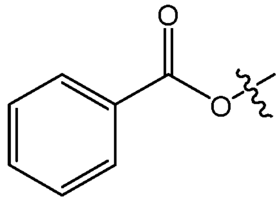

In the present disclosure, a sulfonamide group may be $-SO_2NR'R''$, wherein the R' and R" are the same as or different from each other, and may be each independently selected from the group consisting of hydrogen; deuterium; halogen; a nitrile group; a substituted or unsubstituted monocyclic or polycyclic cycloalkyl group having 3 to 30 carbon atoms; a substituted or unsubstituted straight-chain or branched-chain alkyl group having 1 to 30 carbon atoms; a substituted or unsubstituted monocyclic or polycyclic aryl group having 6 to 30 carbon atoms; and a substituted or unsubstituted monocyclic or polycyclic heteroaryl group having 2 to 30 carbon atoms.

In the present disclosure, an alkyl group may be straight-chain or branched-chain, and the carbon number of the straight-chain alkyl group is not particularly limited, but may be 1 to 20. Also, the carbon number of the branched-chain alkyl group is 3 to 20. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto. The alkyl group may be substituted or unsubstituted, and if substituted, examples of the substituent are as described above.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto. The cycloalkyl group may be substituted or unsubstituted, and if substituted, examples of the substituent are as described above.

In the present disclosure, an alkoxy group is a functional group in which the above-mentioned alkyl group is bonded to one end of an ether group(-O-), and the description of the alkyl group as defined may be applied, except that each of these are functional groups is bonded to an ether group (-O-). For example, the alkoxy group may be straight chain, branched chain, or cyclic chain. The carbon number of the alkoxy group is not particularly limited, but may be 1 to 20. Specific examples thereof include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, cycloheptoxy, benzyloxy, p-methylbenzyloxy, and the like, but are not limited thereto. The alkoxy group may be substituted or unsubstituted, and if substituted, examples of the substituent are as described above.

In the present disclosure, an amine group may be selected from the group consisting of $-NH_2$, a monoalkylamine group, a dialkylamine group, an N-alkylarylamine group, a monoarylamine group, a diarylamine group, an N-arylheteroarylamine group, an N-alkylheteroarylamine group, a monoheteroarylamine group and a diheteroarylamine, and the carbon number thereof is not particularly limited, but may be 1 to 30. Specific examples of the amine group include a methylamine group, a dimethylamine group, an ethylamine group, a diethylamine group, a phenylamine group, a naphthylamine group, a biphenylamine group, an anthracenylamine group, a 9-methyl-anthracenylamine group, a diphenylamine group, a ditolylamine group, an N-phenylbiphenylamine group, an N-phenylnaphthylamine group, an N-biphenylnaphthylamine group, a ditolylamine group, an N-phenyltolylamine group, a triphenylamine group, an N-naphthylfluorenylamine group, an N-phenylphenanthrenylamine group, an N-biphenylphenanthrenylamine group, an N-phenylfluorenylamine group, an N-phenylterphenylamine group, an N-phenanthrenylfluorenylamine group, an N-biphenylfluorenylamine group, and the like, but are not limited thereto. The amine group may be substituted or unsubstituted, and if substituted, examples of the substituent are as described above.

In the present disclosure, an N-alkylarylamine group means an amine group in which N of the amine group is substituted with an alkyl group and an aryl group.

In the present disclosure, an N-arylheteroarylamine group means an amine group in which N of the amine group is substituted with an aryl group and a heteroaryl group.

In the present disclosure, an N-alkylheteroarylamine group means an amine group in which N of the amine group is substituted with an alkyl group and a heteroaryl amine group.

In the present disclosure, an alkyl group in the monoalkylamine group, dialkylamine group, N-arylalkylamine group, alkylthioxy group, alkylsulfoxy group, and N-alkylheteroarylamine group are the same as examples of the alkyl group as defined above. Specifically, the alkylthioxy group includes a methylthioxy group, an ethylthioxy group, a tert-butylthioxy group, a hexylthioxy group, an octylthioxy group, and the like, and the alkyl sulfoxy group includes a mesyl group, an ethyl sulfoxy group, a propyl sulfoxy group, a butyl sulfoxy group, and the like, but are not limited thereto.

In the present disclosure, an alkenyl group may be straight-chain or branched-chain, and the carbon number thereof is not particularly limited, but may be 2 to 40. The description of the alkyl group as defined above may be applied except that the alkenyl group includes at least one unsaturated bond (double bond or triple bond) in the functional group. According to one embodiment, the carbon number of the alkenyl group is 2 to 20. According to another embodiment, the carbon number of the alkenyl group is 2 to 10. According to another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto. The alkenyl group may be substituted or unsubstituted, and if substituted, examples of the substituent are as described above.

In the present disclosure, a cycloalkenyl group may have 3 to 60 carbon atoms. According to one embodiment, the carbon number of the cycloalkenyl group is 3 to 30. According to another embodiment, the carbon number of the cycloalkenyl group is 3 to 20. According to another embodiment, the carbon number of the cycloalkenyl group is 3 to 6. The description of the cycloalkyl group as defined above may be applied except that the cycloalkenyl group includes at least one unsaturated bond (double bond or triple bond) in the functional group. The cycloalkenyl group may be substituted or unsubstituted, and if substituted, examples of the substituent are as described above.

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto. The silyl group may be substituted or unsubstituted, and if substituted, examples of the substituent are as described above.

In the present disclosure, a boron group specifically includes a trimethyl boron group, a triethyl boron group, a t-butyldimethyl boron group, a triphenyl boron group, and a phenyl boron group, but is not limited thereto. The boron group may be substituted or unsubstituted, and if substituted, examples of the substituent are as described above.

In the present disclosure, a phosphine oxide group specifically includes a diphenylphosphine oxide group, a dinaphthylphosphine oxide, and the like, but are not limited thereto. The phosphine oxide group may be substituted or unsubstituted, and if substituted, examples of the substituent are as described above.

In the present disclosure, an aryl group is not particularly limited, but may have 6 to 60 carbon atoms, and may be a monocyclic aryl group or a polycyclic aryl group. According to one embodiment, the carbon number of the aryl group is 6 to 30. According to another embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthrenyl group, a pyrenyl group, a perylenyl group, a chrysenyl group, or the like, but is not limited thereto. The aryl group may be substituted or unsubstituted, and if substituted, examples of the substituent are as described above.

In the present disclosure, an aryl group in the monoarylamine group, diarylamine group, aryloxy group, arylthioxy group, arylsulfoxy group, N-arylalkylamine group, N-arylheteroarylamine group and arylphosphine group is the same as the example of the aryl group as defined above. Specifically, the the aryloxy group includes phenoxy group, p-toryloxy group, m-toryloxy group, 3,5-dimethyl-phenoxy group, 2,4,6-trimethylphenoxy group, p-tert-butylphenoxy group, 3-biphenyloxy group, 4-biphenyloxy group, 1-naphthyloxy group, 2-naphthyloxy group, 4-methyl-1-naphthyloxy group, 5-methyl-2-naphthyloxy group, 1-anthryloxy group, 2-anthryloxy group, 9-anthryloxy group, 1-phenanthryloxy group, 3-phenanthryloxy group, 9-phenanthryloxy group, or the like, the arylthioxy group includes phenylthioxy group, 2-methylphenylthioxy group, and 4-tert-butylphenylthioxy group, or the like, and the aryl sulfoxy group includes benzene sulfoxy group and p-toluene sulfoxy group, or the like, but are not limited thereto.

In the present disclosure, a heterocyclic group is a heterocyclic group containing one or more of O, N, Se and S as a heteroatom, and the carbon number thereof is not particularly limited, but may be 2 to 60. Examples of the heterocyclic group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, a triazole group, an acridyl group, a pyridazine group, a pyrazinyl group, a quinolinyl group, a quinazoline group, a quinoxalinyl group, a

phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, a thiazolyl group, an isoxazolyl group, an oxadiazolyl group, a thiadiazolyl group, a benzothiazolyl group, a phenothiazinyl group, a dibenzofuranyl group, and the like, but are not limited thereto.

In the present disclosure, a heteroaryl group includes at least one heteroatom (i.e. atom other than carbon). Specifically, the heteroatom may include at least one atom selected from the group consisting of O, N, Se, and S. The carbon number thereof is not particularly limited, but may be 2 to 60 carbon atoms. The heteroaryl group may be monocyclic or polycyclic. Examples of the heteroaryl group include a thiophene group, a furanyl group, a pyrrole group, an imidazolyl group, a thiazolyl group, an oxazolyl group, an oxadiazolyl group, a pyridyl group, a bipyridyl group, a pyrimidyl group, a triazinyl group, a triazolyl group, an acridyl group, a pyridazinyl group, a pyrazinyl group, a quinolinyl group, a quinazolinyl group, a quinoxalinyl group, a phthalazinyl group, a pyrido pyrimidyl group, a pyrido pyrazinyl group, a pyrazino pyrazinyl group, an isoquinolinyl group, an indolyl group, a carbazolyl group, a benzoxazolyl group, a benzimidazolyl group, a benzothiazolyl group, a benzocarbazolyl group, a benzothiophene group, a dibenzothiophene group, a benzofuranyl group, a phenanthroline group, a thiazolyl group, an isoxazolyl group, an oxadiazolyl group, a thiadiazolyl group, a benzothiazolyl group, a phenothiazinyl group, an aziridyl group, an azaindolyl group, an isoindolyl group, an indazolyl group, a purine group, a pteridine group, a beta-carbolyl group, a naphthyridine group, a terpyridyl group, a phenazinyl group, an imidazopyridyl group, a pyropyridyl group, an azepine group, a pyrazolyl group, a dibenzofuranyl group, and the like, but are not limited thereto. The heteroaryl group may be substituted or unsubstituted, and if substituted, examples of the substituent are as described above.

In the present disclosure, examples of the heteroaryl group in the monoheteroarylamine group, diheteroarylamine group, N-arylheteroarylamine group, and N-alkylheteroarylamine group may be selected from examples of the heteroaryl group as defined above.

In the present disclosure, an alkylene group is a divalent functional group derived from an alkane, for example, a straight chain or branched chain, and may be a methylene group, an ethylene group, a propylene group, an isobutylene group, a sec-butylene group, a tert-butylene group, a pentylene group, a hexylene group, and the like. The alkylene group may be substituted or unsubstituted, and if substituted, examples of the substituent are as described above.

In the present disclosure, a halo alkylene group means a functional group in which the above-mentioned alkylene group is substituted with a halogen group, and an example thereof may be perfluoropropane-2,2-diyl, and the like.

In the present disclosure, an alkenylene group is a divalent functional group derived from an alkenyl, and the description of the alkenyl group as defined above may be applied except that it is a divalent functional group. The alkenylene group may be substituted or unsubstituted, and if substituted, examples of the substituent are as described above.

In the present disclosure, a cycloalkylene group is a divalent functional group derived from a cycloalkane, and the description of the cycloalkyl group as defined above may be applied except that it is a divalent functional group. The cycloalkylene group may be substituted or unsubstituted, and if substituted, examples of the substituent are as described above.

In the present disclosure, a cycloalkenylene group is a divalent functional group derived from a cycloalkenyl, and the description of the cycloalkenyl group as defined above may be applied except that it is a divalent functional group. The cycloalkenylene group may be substituted or unsubstituted, and if substituted, examples of the substituent are as described above.

In the present disclosure, an arylene group means an aryl group with two bonding positions, that is, a divalent functional group. The description of the aryl group defined above may be applied, except that each of these is a divalent functional group. The arylene group may be substituted or unsubstituted, and if substituted, examples of the substituent are as described above.

1. Salt **compound**

According to one embodiment of the present disclosure, there can be provided a salt compound comprising an anion represented by the following Chemical Formula 1, and a cation represented by the following Chemical Formula 2:

[Chemical Formula 1]

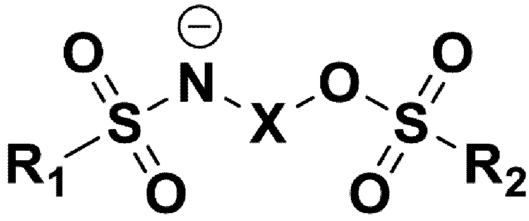

[Chemical Formula 2]

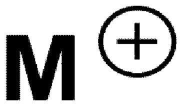

wherein, in Chemical Formulas 1 and 2, $R_1$ and $R_2$ are the same as or different from each other, and are each independently one of an alkyl group, an alkenyl group, a cycloalkyl group, a cycloalkenyl group, an aryl group, a halogen, or a combination thereof, X is one of a direct bond, an alkylene group, an alkenylene group, a cycloalkylene group, a cycloalkenylene group, an arylene group, or a combination thereof, and $M^+$ is either an onium ion or a metal ion.

The combination of functional groups in the $R_1$, $R_2$, and X means a combinatorial functional group containing two or more functional groups together, and may include, for example, an arylalkyl group containing both an aryl group and an alkyl group, and the like.

At least one or more hydrogen atoms included in the alkyl group, alkenyl group, cycloalkyl group, cycloalkenyl group, aryl group, alkylene group, alkenylene group, cycloalkylene group, cycloalkenylene group, and arylene group are each independently substituted or unsubstituted with one or more substituents selected from the group consisting of deuterium; halogen; a cyano group; a nitro group; a hydroxyl group; a carbonyl group; an ester group; an imide group; an amide group; an amino group; a carboxyl group; a sulfonic acid group; a sulfonamide group; a phosphine oxide group; an alkoxy group; an alkylcarbonyl group; an alkoxycarbonyl group; a sulfonyloxy group; an aryloxy group; an alkylthioxy group; an arylthioxy group; an alkyl sulphoxy group; an aryl sulfoxy group; a silyl group; and a boron group,

at least one or more carbon atoms included in the alkyl group, alkenyl group, cycloalkyl group, cycloalkenyl group, aryl group, alkylene group, alkenylene group, cycloalkylene group, cycloalkenylene group, and arylene group are each independently substituted or unsubstituted with a hetero element,

the alkyl group, alkenyl group, alkylene group, and alkenylene group are each independently straight chain or branched chain, and

the cycloalkyl group, cycloalkenyl group, aryl group, cycloalkylene group, cycloalkenylene group, and arylene group are each independently monocyclic or polycyclic.

The present inventors have found through experiments that as the salt compound of one embodiment is further imparted with polarity by the sulfonyloxy group ($-SO_3-$) included in the anion structure, as represented by Chemical Formula 1, it shortens the diffusion length of anions generated from the quencher during exposure, thereby securing high contrast, improving the marginal resolving power, and obtaining low sensitivity, LER values and high process margins, and completed the present disclosure.

On the other hand, if a sulfonyl group ($-SO_2-$) is included instead of the above-mentioned sulfonyloxy group ($-SO_3-$), there is a limitation that the diffusion length of anions generated from the quencher during exposure is difficult to be shortened to a sufficient level while polarity is reduced due to the decrease in oxygen atoms.

Moreover, in the anion structure represented by Chemical Formula 1, when the sulfonyloxy group($-SO_3-$) and the nitrogen(N) atom are bonded via a mediating functional group represented by X, rather than when they are bonded directly, the salt compound of one embodiment not only improves miscibility with other constituent components forming the resist and improves the uniformity of distribution within the resist thin film, but also increases the acid diffusion suppressing effect and thus can achieve the effect of improving the contrast between the exposed area/non-exposed area and improving the line width roughness.

On the other hand, when the above-mentioned sulfonyloxy group($-SO_3-$) and a nitrogen(N) atom are directly bonded, or when two sulfonyl groups($-SO_2-$) are each directly bonded to a nitrogen(N) atom, there may be problems that the acid diffusion suppressing efficiency decreases while the polarity decreases due to a decrease in oxygen atoms, the miscibility with resist components decreases, and the distribution uniformity within the thin film deteriorates, whereby

contrast between the exposed area and the non-exposed area becomes insufficient, process margin decreases, and line width roughness increases.

Specifically, in the anion represented by Chemical Formula 1, X may be one of a direct bond, an alkylene group, an alkenylene group, a cycloalkylene group, a cycloalkenylene group, an arylene group, or a combination thereof. Specifically, in Chemical Formula 1, X may be one of an alkylene group having 1 to 30 carbon atoms, an alkenylene group having 1 to 30 carbon atoms, a cycloalkylene group having 3 to 30 carbon atoms, a cycloalkenylene group having 3 to 30 carbon atoms, or an arylene group having 6 to 30 carbon atoms.

The X may specifically be an alkylene group or an alkenylene group, and in this case, the reactivity is excellent and thus a high yield can be obtained.

The alkylene group having 1 to 30 carbon atoms may be an alkylene group having 1 to 20 carbon atoms, or 1 to 10 carbon atoms, or 1 to 8 carbon atoms, or 1 to 3 carbon atoms, or 2 to 6 carbon atoms. More specifically, in Chemical Formula 1, X is an alkylene group having 2 to 3 carbon atoms.

Specific examples of X in Chemical Formula 1 are not particularly limited, but it may be, for example, ethylene or propylene. As X in Chemical Formula 1 satisfies an alkylene group having 2 to 3 carbons, the crystallinity during purification is improved and a high purity substance can be obtained in high yield. On the other hand, when X in Chemical Formula 1 is an alkylene group having more than 3 carbon atoms, it is difficult to purify due to side reactions and gel-like properties, which may cause a problem that the yield decreases.

Further, specific examples of X in Chemical Formula 1 include butylene, pentylene, hexylene, heptylene, octylene, and the like.

Meanwhile, in the anion represented by Chemical Formula 1, $R_1$ and $R_2$ are the same as or different from each other, and may be each independently one of an alkyl group, an alkenyl group, a cycloalkyl group, a cycloalkenyl group, an aryl group, a halogen, or a combination thereof.

Specifically, in Chemical Formula 1, $R_1$ may be an alkyl group having 1 to 30 carbon atoms in which the hydrogen atom is unsubstituted. Specific examples of the alkyl group having 1 to 30 carbon atoms in which the hydrogen atom is unsubstituted are not particularly limited, but it may include, for example, methyl, ethyl, or propyl.

Further, in Chemical Formula 1, $R_1$ may be an aryl group having 6 to 30 carbon atoms in which the hydrogen atom is unsubstituted. Specific examples of the aryl group having 6 to 30 carbon atoms in which the hydrogen atom is unsubstituted are not particularly limited, but it may include, for example, phenyl.

Further, in Chemical Formula 1, $R_1$ may be halogen. Specific examples of the halogen are not particularly limited, but it may include, for example, fluorine, chlorine, bromine, and iodine.

Further, in Chemical Formula 1, $R_1$ may be a cycloalkyl group having 3 to 30 carbon atoms in which the hydrogen atom is unsubstituted. Specific examples of the cycloalkyl group having 3 to 30 carbon atoms in which the hydrogen atom is unsubstituted are not particularly limited, but it may include, for example, cyclohexyl or adamantyl.

Further, in Chemical Formula 1, $R_1$ may be an alkyl group having 1 to 30 carbon atoms in which at least one hydrogen atom is substituted with halogen, or an aryl group having 6 to 30 carbon atoms in which at least one hydrogen atom is substituted with halogen, or an aryl group having 1 to 30 carbon atoms in which at least one hydrogen atom is substituted with a halogenated alkyl group. Thereby, the absorption efficiency of the exposure light source can be increased, and the resolving power can be improved.

Specific examples of the alkyl group having 1 to 30 carbon atoms in which at least one hydrogen atom is substituted with halogen are not particularly limited, but it may be, for example, trifluoromethyl. Specific examples of the aryl group having 6 to 30 carbon atoms in which at least one hydrogen atom is substituted with halogen are not particularly limited, but it may include, for example, one of 4-fluorophenyl, 4-bromophenyl, 4-iodophenyl, 2,5-difluorophenyl, 2,4-difluorophenyl, 4-(trifluoromethyl)phenyl, or 1,2,3,4,5-pentafluorophenyl.

Specifically, in Chemical Formula 1, $R_1$ may be one of an alkyl group having 1 to 30 carbon atoms in which 2 or more and 6 or less hydrogen atoms are substituted with halogen, or an aryl group having 6 to 30 carbon atoms in which 2 or more and 6 or less hydrogen atoms are substituted with halogen. Thereby, the absorption efficiency of the exposure light source is increased in the exposed area and the acid diffusion suppressing efficiency is increased, thereby exhibiting the effect of improving the contrast between the exposed area and the non-exposed area and increasing the resolving power.

Specific examples of the alkyl group having 1 to 30 carbon atoms in which 2 or more and 6 or less hydrogen atoms are substituted with halogen are not particularly limited, but it may include, for example, one of trifluoromethyl.

Specific examples of the aryl group having 6 to 30 carbon atoms in which 2 or more and 6 or less hydrogen atoms are substituted with halogen are not particularly limited, but it may include, for example, one of 4-trifluoromethylphenyl, 4-fluorophenyl, 2,5-difluorophenyl, 2,4-difluorophenyl, 4-(trifluoromethyl)phenyl, or 1,2,3,4,5-pentafluorophenyl.

Specifically, in Chemical Formula 1, $R_1$ may be one of methyl, ethyl, propyl, halogen, trifluoromethyl, cyclohexyl, adamantyl, phenyl, 4-fluorophenyl, 4-bromophenyl, 4-iodophenyl, 2,4-difluorophenyl, 2,5-difluorophenyl, 4-(trifluoromethyl)phenyl, or 1,2,3,4,5-pentafluorophenyl.

Meanwhile, in Chemical Formula 1, $R_2$ may be a substituted or unsubstituted aryl group. When the unsaturated aromatic group $R_2$ in Chemical Formula 1 is selected and substituted, the absorption of electron beams or EUV photons

increases, so that more secondary electrons can be generated within the resist thin film, and thus the sensitivity can be improved.

Specifically, the $R_2$ may be one of an aryl group in which at least one hydrogen atom is substituted with halogen, an aryl group in which a hydrogen atom is unsubstituted, or an aryl group in which at least one hydrogen atom is substituted with a heteroalkyl group.

Preferably, the $R_2$ may be an aryl group in which at least one hydrogen atom is substituted with halogen. In particular, iodine among halogen elements is preferred in terms of improving secondary electron generation efficiency. Specific examples of the aryl group in which at least one hydrogen atom is substituted with halogen are not particularly limited, but it may include, for example, 4-fluorophenyl, 4-bromophenyl, 4-iodophenyl, 2,5-difluorophenyl, 2,4-difluorophenyl, or 1,2,3,4,5-pentafluorophenyl.

Meanwhile, the $R_2$ may be an aryl group in which a hydrogen atom is unsubstituted. Specific examples of the aryl group in which the hydrogen atom is unsubstituted are not particularly limited, but it may be, for example, 1-naphthyl, or 2-naphthyl, 4-pyridinyl, 6-benzooxazolyl, 5-phthalanyl, 1,4-benzodioxan-6-yl, acetyl-4-piperidinyl, or tert-butoxycarbonyl-4-piperidinyl.

Further, the $R_2$ may be an aryl group in which at least one hydrogen atom is substituted with a heteroalkyl group. Specific examples of the heteroalkyl group include an alkoxy group, and specific examples of the aryl group in which at least one hydrogen atom is substituted with a heteroalkyl group are not particularly limited, but it may include, for example, 4-methoxyphenyl.

Meanwhile, the $R_2$ may be one of an alkyl group, an alkenyl group, a cycloalkyl group, a cycloalkenyl group, or a combination thereof. More specifically, the alkyl group, alkenyl group, cycloalkyl group, and cycloalkenyl group may be a group in which a hydrogen atom is unsubstituted. Specific examples of the alkyl group, alkenyl group, cycloalkyl group, cycloalkenyl group, or a combination thereof in which the hydrogen atom is unsubstituted are not particularly limited, but it may include, for example, methyl, ethyl, propyl, trifluoromethyl, cyclohexyl, adamantyl, or 1,4-dioxan-2-ylethyl.

Further, the $R_2$ may be one of an alkyl group, an alkenyl group, a cycloalkyl group, a cycloalkenyl group, or a combination thereof, in which at least one hydrogen atom is substituted with a heteroalkyl group. Specific examples of the heteroalkyl group include an alkoxy group.

More specifically, in Chemical Formula 1, $R_2$ may be one of methyl, ethyl, propyl, trifluoromethyl, cyclohexyl, adamantyl, acetyl-4-piperidinyl, tert-butoxycarbonyl-4-piperidinyl, 4-fluorophenyl, 4-bromophenyl, 4-iodophenyl, 2,5-difluorophenyl, 2,4-difluorophenyl, 1,2,3,4,5-pentafluorophenyl, 4-methoxyphenyl, 1-naphthyl, 2-naphthyl, 4-pyridinyl, 6-benzooxazolyl, 5-phthalanyl, 1,4-dioxan-2-ylethyl, or 1,4-benzodioxan-6-yl.

Specific examples of the anion represented by Chemical Formula 1 are not particularly limited, but it may be, for example, any one selected from the group consisting of:

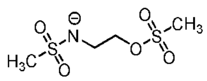 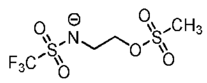 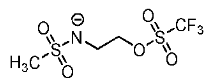 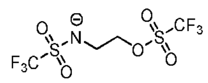

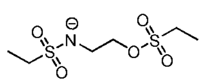 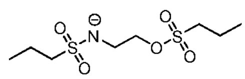 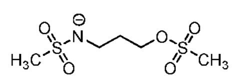 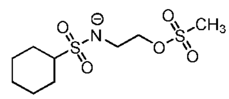

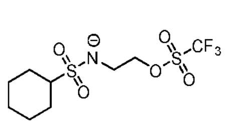 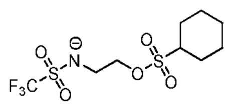 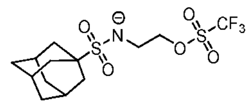 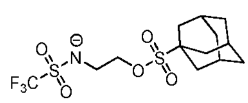

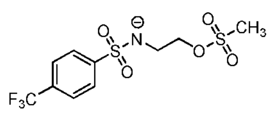 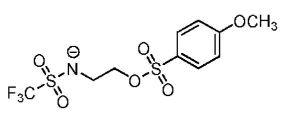 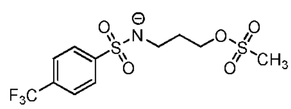 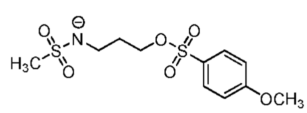

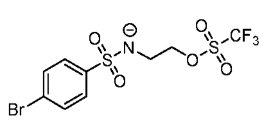 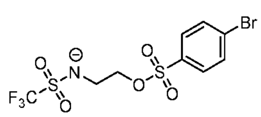 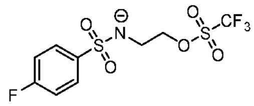 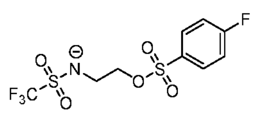

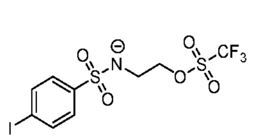
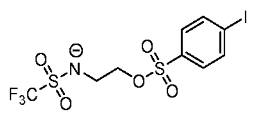
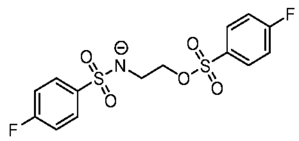
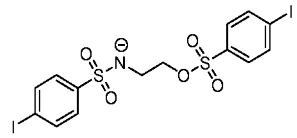

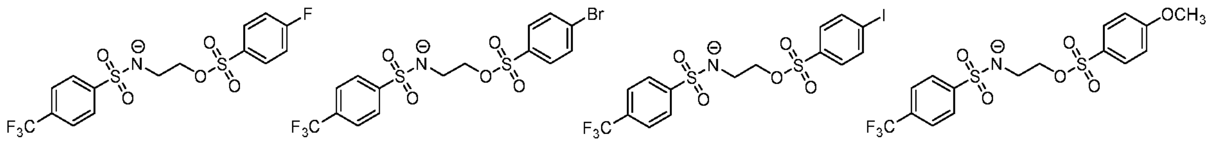

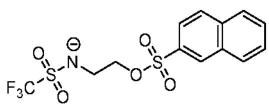
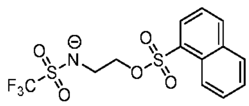
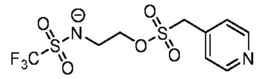
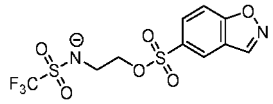

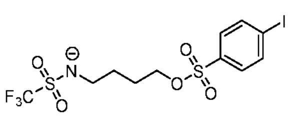
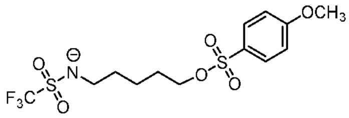
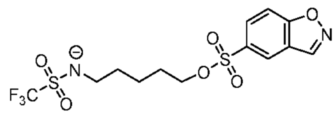

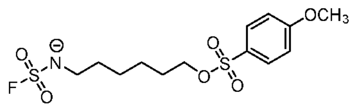
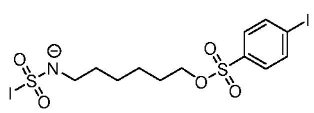
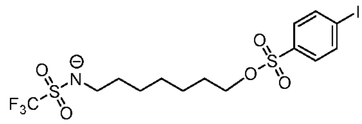

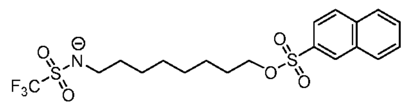

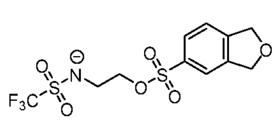
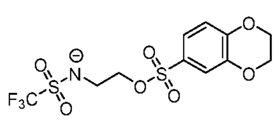
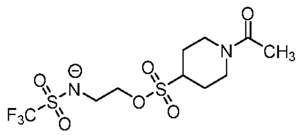
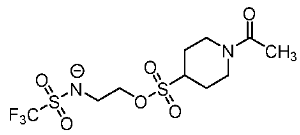

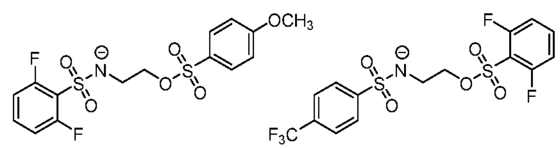
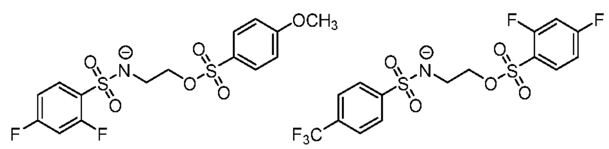

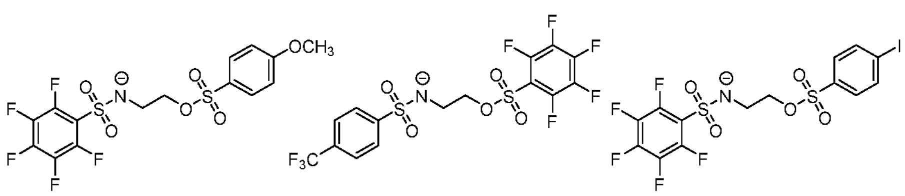
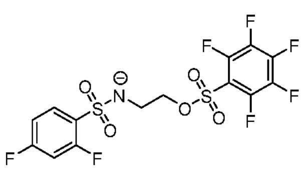

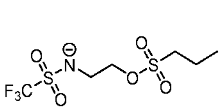
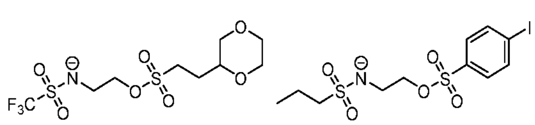
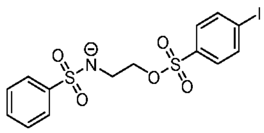.

Meanwhile, in Chemical Formula 2, $M^+$ may be either an onium ion or a metal ion. Specifically, in Chemical

Formula 2, $M^+$ may be one of an onium ion, a sulfonium ion, an iodonium ion, or an ammonium ion.

Specific examples of the cation represented by Chemical Formula 2 are not particularly limited, but may be, for example, any one selected from the group consisting of:

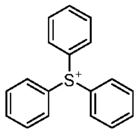 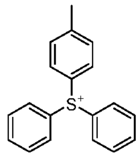 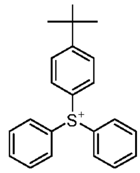 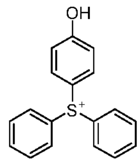

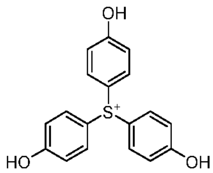 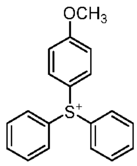 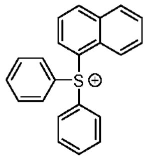 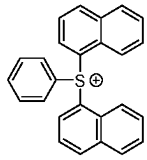

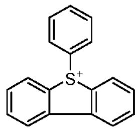 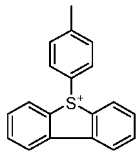 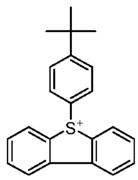 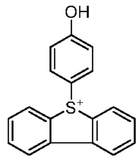

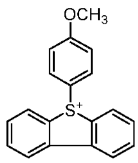 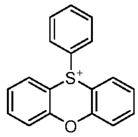 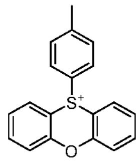 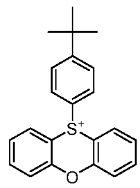

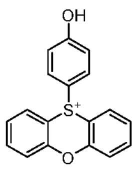 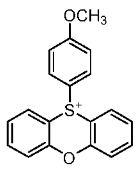 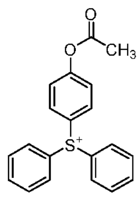 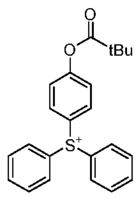

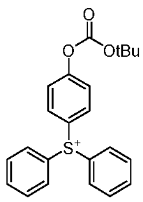 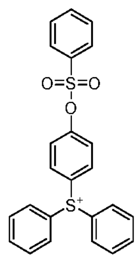 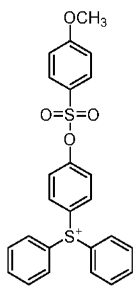 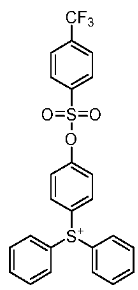

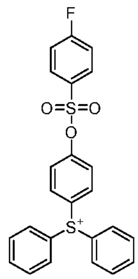
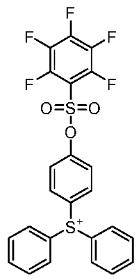
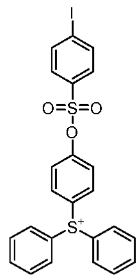
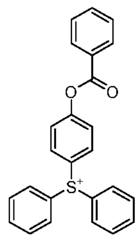

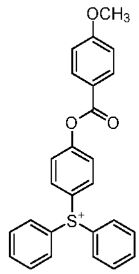
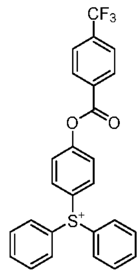
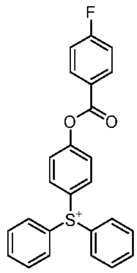
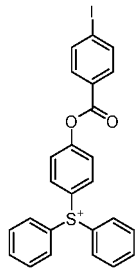

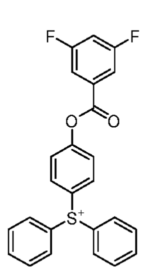
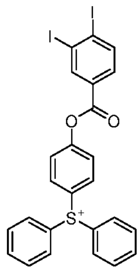
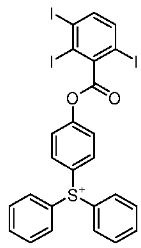
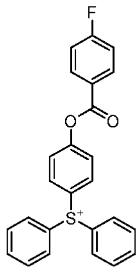

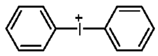
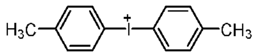
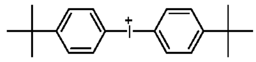
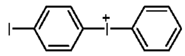.

The specific example of the method for synthesizing the salt compound is not particularly limited, but as an example, it can be synthesized according to the following Reaction Scheme 1.

[Reaction Scheme 1]

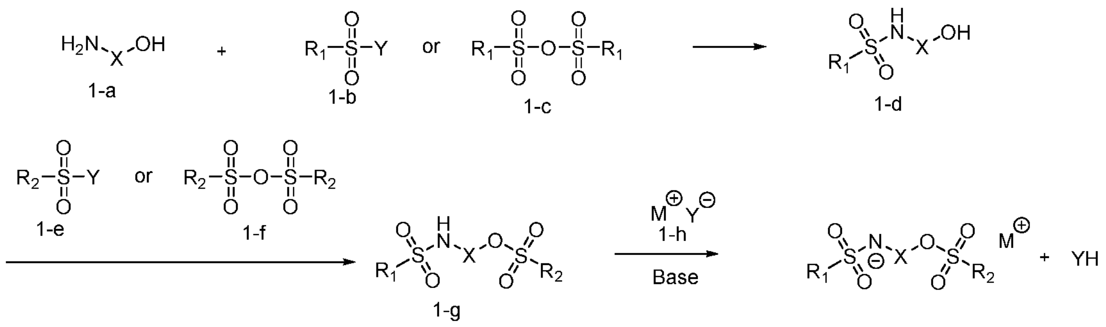


in Reaction Scheme 1, $R_1$, $R_2$, X, and M are the same as $R_1$, $R_2$, X (Chemical Formula 1) and M (Chemical Formula 2). Y is a counter anion, and Base is a base.

First, Compound 1-a may be reacted with Compound 1-b or Compound 1-c to obtain Compound 1-d. Compound 1-d may be again reacted with Compound 1-e or Compound 1-f to obtain Compound 1-g, wherein Compounds 1-e and 1-f may be the same as or different from Compounds 1-b and 1-c. Compound 1-g may be reacted with Compound 1-h to obtain Compound A.

Among the above reactions, the nucleophilic reaction of the amine group occurs more readily than the nucleophilic reaction of the alcohol group in the synthesis reaction for obtaining Compound 1-d, however, in order to

optionally induce an amine group substitution reaction, Compound 1-b or Compound 1-c may be slowly added dropwise while maintaining the reaction temperature at -40°C or less and -78°C or more. If the structures of Compound 1-b and Compound 1-e are the same, the reaction temperature is sufficient to slowly drop either Compound 1-b or 1-e at 0°C and then slowly raise the temperature to room temperature, and the method for obtaining Compound 1-g may also be obtained by a method similar thereto.

The method of reacting Compound 1-g and Compound 1-h to obtain a salt compound is not particularly limited, and may be obtained by dissolving Compound 1-g in an appropriate organic solvent and water in the presence of an appropriate alkali metal hydroxide, adding Compound 1-h thereto, and then stirring the mixture so that the organic layer and the water layer are well mixed.

The alkali metal hydroxide in the above reaction is not particularly limited, general sodium hydroxide, potassium hydroxide, and the like may be used, and the amount used may be 1 mole or more and 2 moles or less with respect to 1 mole of Compound 1-g.

As the organic solvent in the reaction, a solvent that can well dissolve the salt compound that is the product, such as dichloromethane or chloroform, may be used, and the amount used may be 15 parts by mass or more and 20 parts by mass or less with respect to Compound 1-g.

The amount of Compound 1-h used in the above reaction may generally be 1 mole or more and 3 moles or less with respect to 1 mole of Compound 1-g, the reaction time varies depending on reactivity, reaction concentration, and the like, but may generally be 10 hours or more and 24 hours or less, and the reaction temperature may be 20°C or more and 50°C or less.

After the reaction is completed, the aqueous layer and the organic layer are separated, and Compound A dissolved in the organic layer may be purified by a purification method selected from conventionally known purification methods such as solvent extraction, concentration, chromatography, crystallization, recrystallization, and the like, wherein the purification methods may be used alone or in combination. This method can be applied to purify not only the final compound A, but also Compound 1-d, Compound 1-g, and Compound 1-h synthesized in each step.

## 2. Quencher

Meanwhile, according to another embodiment of the present disclosure, there can be provided a quencher comprising the salt compound of the one embodiment.

The details regarding the salt compound includes those described above regarding the one embodiment.

The quencher is a material that can adjust the acid diffusion distance, and functions as a quencher to suppress acid diffusion in the non-exposed area, and photolyze and function as acids in the exposed area to maintain the acid concentration in the exposed area relatively high, thereby increasing the acid distribution contrast between the exposed area and the non-exposed area and thus improving lithographic characteristics.

As the quencher used in the present disclosure, in addition to the above salt compounds, a conventional quencher for photoresist can be further mixed and used without limitation. The conventional quencher for photoresist may include, for example, primary, secondary and tertiary aliphatic amines, mixed amines, aromatic amines, heterocyclic amines, a nitrogen-containing compound having a carboxyl group, a nitrogen-containing compound having a sulfonyl group, a nitrogen-containing compound having a hydroxy group, a nitrogen-containing compound having a hydroxyphenyl group, an alcoholic nitrogen-containing compound, amides, imides, carbamates, and the like.

## 3. Photoresist composition

Meanwhile, according to another embodiment of the present disclosure, there can be provided a photoresist composition comprising: the salt compound of the one embodiment; a photoacid generator; a base polymer; and an organic solvent.

The details regarding the salt compound include those described above regarding the one embodiment. The content of the salt compound or the quencher containing the same may be 0.01 parts by weight or more and 30 parts by weight or less, or 0.1 parts by weight or more and 23 parts by weight or less with respect to 100 parts by weight of the base polymer. If the content of the salt compound or the quencher containing the same is out of the above range, there is a possibility that a large amount of acid is generated, resulting in a pattern with a poor cross section, and the contrast of the pattern may be reduced.

Meanwhile, the base polymer has the property of changing solubility in a developer solution due to the action of acid, and the photosensitive polymer used in a conventional photoresist composition can be used without limitation.

As a specific example, the base polymer may include a repeating unit represented by the following Chemical Formula a or Chemical Formula b. The base polymer may be a homopolymer consisting of one or more repeating units represented by Chemical Formula a, a homopolymer consisting of one or more repeating units represented by Chemical Formula b, a copolymer consisting of two or more repeating units represented by Chemical Formula a, a copolymer

consisting of two or more repeating units represented by Chemical Formula b, or a copolymer consisting of one or more repeating units represented by Chemical Formula a and one or more repeating units represented by Chemical Formula b.

[Chemical Formula a]

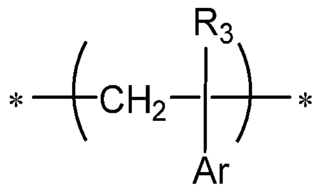

wherein, in Chemical Formula a, $R_3$ may be hydrogen or a methyl group, and Ar may be an aryl group in which at least one hydrogen atom is substituted or unsubstituted. The aryl group may be monocyclic or polycyclic. Examples of the substituents are not particularly limited, and it may include, for example, at least one substituent selected from the group consisting of deuterium; halogen; a cyano group; a nitro group; a hydroxyl group; a carbonyl group; an ester group; an imide group; an amide group; an amino group; a carboxyl group; a sulfonic acid group; a sulfonamide group; a phosphine oxide group; an alkoxy group; an alkylcarbonyl group; an alkoxycarbonyl group; a sulfonyloxy group; an aryloxy group; an alkylthioxy group; an arylthioxy group; an alkyl sulphoxy group; an aryl sulfoxy group; a silyl group; and a boron group. More specifically, halogen or a hydroxy group may be used as the substituent.

[Chemical Formula b]

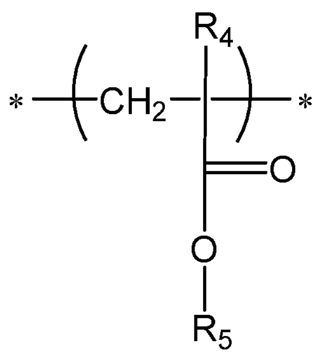

wherein, in Chemical Formula b, $R_4$ is hydrogen or a methyl group, and $R_5$ may be one of an alkyl group, an alkenyl group, a cycloalkyl group, a cycloalkenyl group, or an aryl group. At least one hydrogen atom included in the alkyl group, alkenyl group, cycloalkyl group, cycloalkenyl group, and aryl group may be each independently substituted or unsubstituted with at least one substituent selected from the group consisting of deuterium; halogen; a cyano group; a nitro group; a hydroxyl group; a carbonyl group; an ester group; an imide group; an amide group; an amino group; a carboxyl group; a sulfonic acid group; a sulfonamide group; a phosphine oxide group; an alkoxy group; an alkylcarbonyl group; an alkoxycarbonyl group; a sulfonyloxy group; an aryloxy group; an alkylthioxy group; an arylthioxy group; an alkyl sulphoxy group; an aryl sulfoxy group; a silyl group; and a boron group.

At least one carbon atom included in the alkyl group, alkenyl group, cycloalkyl group, cycloalkenyl group, and aryl group may be each independently substituted or unsubstituted with a hetero element.

The alkyl group and the alkenyl group are each independently straight chain or branched chain, and the cycloalkyl group, cycloalkenyl group, and aryl group may be each independently monocyclic or polycyclic.

More specifically, the base polymer may be a photosensitive polymer (base resin) represented by the following Chemical Formula c.

[Chemical Formula c]

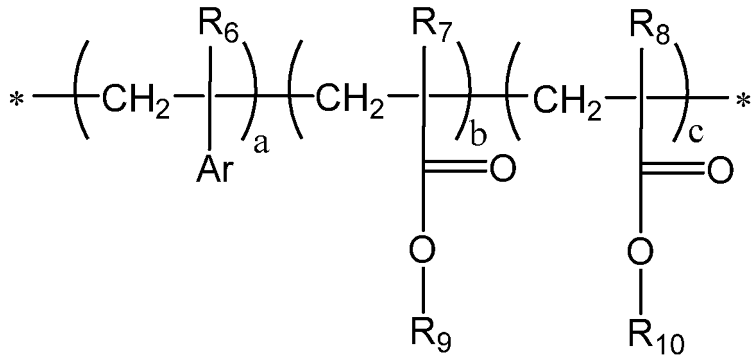

wherein, in Chemical Formula c, $R_6$ to $R_8$ are the same as or different from each other and are each independently hydrogen or a methyl group, Ar is an aryl group in which at least one hydrogen atom is substituted or unsubstituted, $R_9$ and $R_{10}$ are the same as or different from each other, and may be each independently one of an alkyl group, an alkenyl group, a cycloalkyl group, a cycloalkenyl group, or an aryl group. The Ar is the same as Ar in Chemical Formula a, and the $R_9$ and $R_{10}$ are the same as $R_5$ in Chemical Formula b.

More specifically, in Chemical Formula c, Ar may be a monocyclic aryl group having 6 to 11 carbon atoms in which at least one hydrogen atom is substituted with a hydroxy group.

In Chemical Formula c, the $R_9$ may be a monocyclic or polycyclic cycloalkyl group in which at least one carbon atom is substituted with a hetero element, or a polycyclic cycloalkyl group in which at least one hydrogen atom is substituted with a hydroxy group.

In Chemical Formula c, the $R_{10}$ may be a monocyclic or polycyclic cycloalkyl group.

In Chemical Formula c, a, b and c described below the repeating unit are mol% of each repeating unit relative to the total monomers (repeating units) forming the polymer, wherein a may be 10 mol% or more and 60 mol% or less, or 35 mol% or more and 40 mol% or less, b may be 1 mol% or more and 30 mol% or less, or 5 mol% or more and 20 mol% or less, and c may be 30 mol% or more and 60 mol% or less, or 40 mol% or more and 55 mol% or less.

If the mol% of the repeating unit is out of the above range, there is a risk that the physical properties of the photoresist film may deteriorate, the formation of the photoresist film may become difficult, and the contrast of the pattern may deteriorate. Typically, the weight average molecular weight(Mw) of the photosensitive polymer may be 2,000 or more and 20,000 or less, or 3,000 or more and 12,000 or less.

More specific examples of the photosensitive polymer (base resin) represented by Chemical Formula c include a photosensitive polymer (base resin) represented by the following Chemical Formulas d, e, f, and g.

[Chemical Formula d]

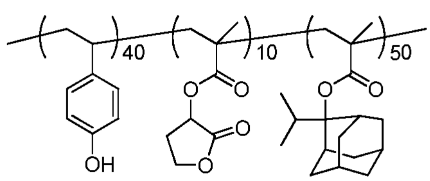

[Chemical Formula e]

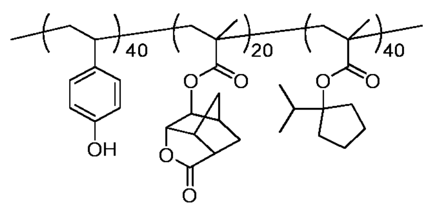

[Chemical Formula f]

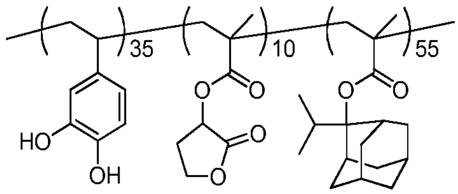

[Chemical Formula g]

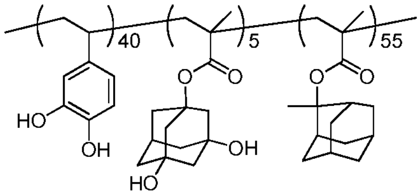

The content of the base polymer may be 2% by weight or more and 30% by weight or less, or 4% by weight or more and 10% by weight or less with respect to the total photoresist composition. If the content of the base polymer is less than 2% by weight, it may be difficult to form a photoresist film and pattern, and if the content exceeds 30% by weight, the thickness distribution of the pattern formed on the wafer may become non-uniform.

The photoacid generator includes onium salt-based, such as sulfonium salts or iodonium salts, diazomethane-based, oxime-based, nitrobenzylsulfonate-based, iminosulfonate-based, disulfone-based acid generators, and the like. These can be used alone or in combination of two or more.

The content of the photoacid generator may be 0.05 parts by weight or more and 30 parts by weight or less, or 0.1 parts by weight or more and 15 parts by weight or less, with respect to 100 parts by weight of the base polymer. If the content of the photoacid generator is less than 0.05 parts by weight with respect to 100 parts by weight of the base polymer, the sensitivity of the photoresist to light may become weaker and the peak diffusion distance may become longer. If the content of the photoacid generator exceeds 30 parts by weight, the photoacid generator absorbs a large amount of far-ultraviolet rays and generates a large amount of acid, which may result in a defective pattern cross section.

As the organic solvent used in the present disclosure, any organic solvent used in conventional photoresist compositions that can easily dissolve the base polymer, the photoacid generator, the salt compound, other additives, and the like can be used without limitation. For example, as the organic solvent, ketones such as cyclohexanone and methyl amyl ketone, alcohols such as 2-methoxybutanol, 3-methyl-3-methoxybutanol, 1-methoxy-2-propanol, and 1-ethoxy-2-propanol, ethers such as propylene glycol monomethyl ether, ethylene glycol monomethyl ether, propylene glycol monoethyl ether, ethylene glycol monoethyl ether, and diethyl glycol dimethyl ether, esters such as propyleneglycol monomethyl etheracetate, propyleneglycol monoethyl etheracetate, ethyl actate, ethyl pyruvate, butyl acetate, methyl 3-methoxypropionate, 3-ethoxypropionate, tert-butylacetate, tert-butyl propionate, propylene glycol mono- tert-butyl ether acetate, lactones such as γ-butyrolactone can be used alone or in combination of two or more, but are not limited thereto. Examples of the organic solvent are not particularly limited, but as an example, any one of PGMEA (propylene glycol monomethyl ether acetate), PGME (propylene glycol monomethyl ether), EL (ethyl lactate), CyH (cyclohexanone), or a mixture thereof, which have the best solubility in an acid diffusion inhibitor, may be used.

The content of the organic solvent is the remainder obtained by excluding the content of the photoresist composition excluding the base polymer, the photoacid generator, the salt compound, and other additives, with respect to 100% by weight of the total photoresist composition.

Examples of the additives are not particularly limited, but for example, an additional resin, a stabilizer, a surfactants, a thickener, a defoaming agent, an adhesion agent, an antioxidant, a dissolution inhibitor, and the like may be used.

According to another embodiment of the present disclosure, there can be provided a method of forming a photoresist pattern comprising the steps of: applying and heating the photoresist composition of the other embodiment to form a photoresist film; exposing the photoresist film; and developing the exposed photoresist film to form a photoresist pattern.

The details regarding the photoresist composition includes those described above regarding the other embodiments.

The pattern forming method may apply a known technique, and for example, includes applying the composition by a spinner, etc., exposing the photoresist film to high-energy rays with a wavelength of 300 nm or less using a

predetermined photo mask, and developing the exposed photoresist film with a conventional developer (e.g., an alkaline aqueous solution such as an aqueous tetramethylammonium hydroxide solution of 0.1% by weight or more and 10% by weight or less).

The high-energy rays with a wavelength of 300 nm or less are not particularly limited, but ultraviolet rays, far-ultraviolet rays, extreme ultraviolet rays (EUV), electron beams, X-rays, excimer lasers, γ-rays, or synchrotron radiation may be exemplified. In order to form fine patterns of 70 nm or less, it is preferable to use an exposure device equipped with a source of short-wavelength high-energy rays, such as an ArF excimer laser, a KrF excimer laser, or an EUV.

More specifically, the high-energy exposure source can be selected from among KrF with a 248nm wavelength, ArF with a 193nm wavelength, e-beam, and EUV with a 13.5nm wavelength.

**[Advantageous Effects]**

According to the present disclosure, there can be provided a salt compound which makes it possible to obtain a photoresist composition, that has improved polarity and thus shortens the diffusion length of anions generated during exposure, ensures high contrast, improves the marginal resolving power, and further has low sensitivity, LER values and high process margins, and a quencher and a photoresist composition comprising the same.

**[DETAILED DESCRIPTION OF THE EMBODIMENTS]**

Below, a preferred example is presented to help the understanding of the present disclosure. However, the following Examples are provided for easier understanding of the present disclosure, and the present disclosure is not limited thereto.

**<Example 1: Preparation of salt compound>**

The structures of the salt compounds obtained in Examples 1-1 to 1-12 and Comparative Examples 1-1 to 1-3 are shown in Table 1 below.

[Table 1] Structure of novel photosensitive salt compound

| Item | Structure |
|---|---|
| Salt compound 1 | 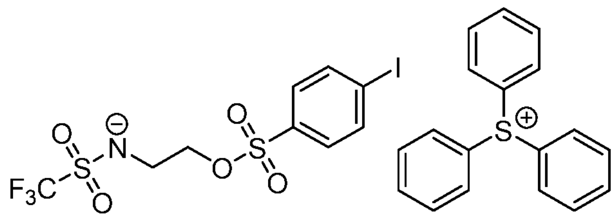 |
| Salt compound 2 | 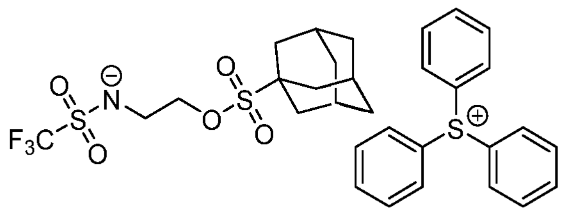 |
| Salt compound 3 | 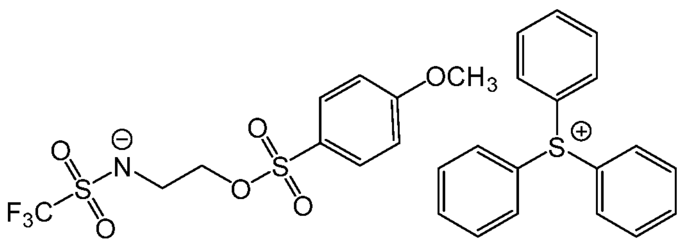 |

(continued)

| Item | Structure |
|---|---|
| Salt compound 4 | 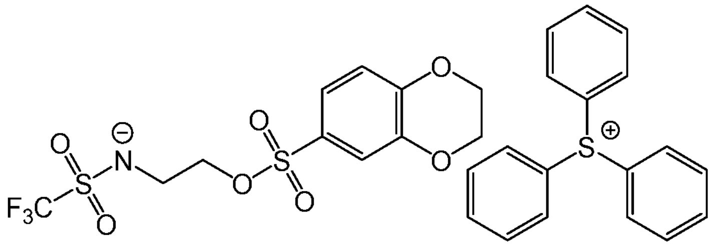 |
| Salt compound 5 | 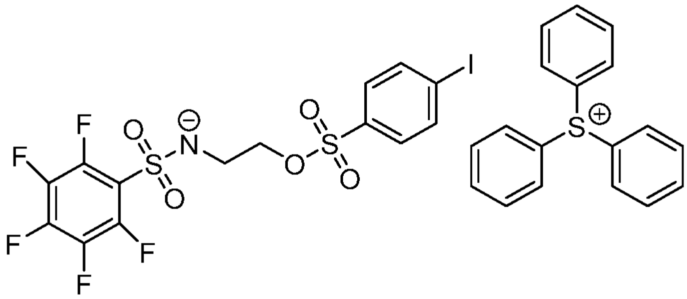 |
| Salt compound 6 | 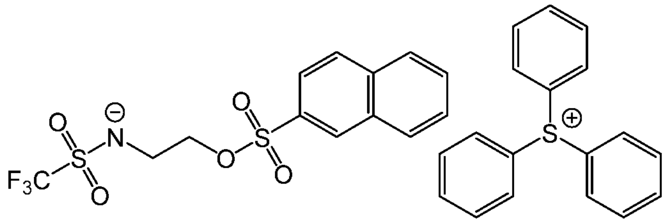 |
| Salt compound 7 | 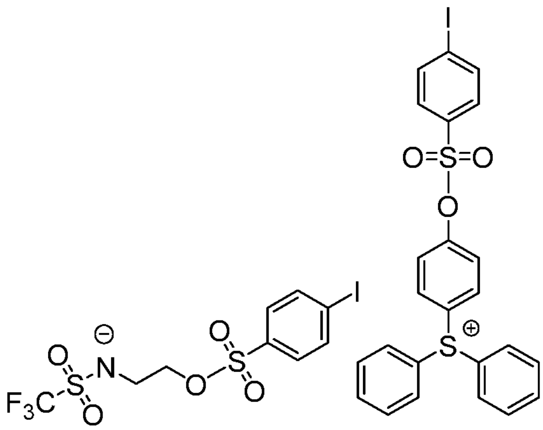 |
| Salt compound 8 | 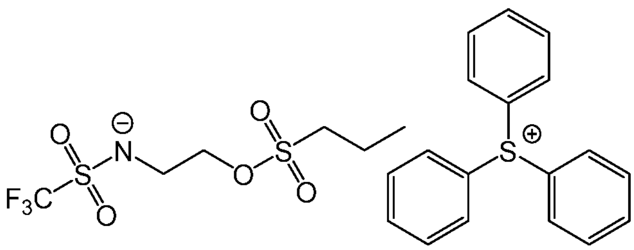 |

(continued)

| Item | Structure |
|---|---|
| Salt compound 9 | 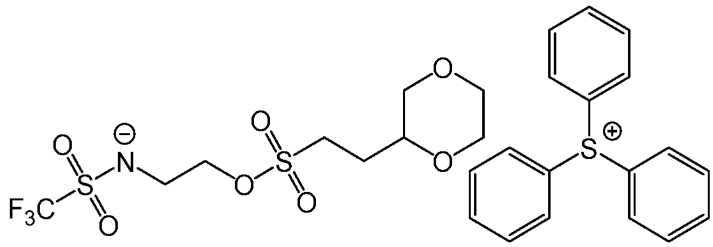 |
| Salt compound 10 | 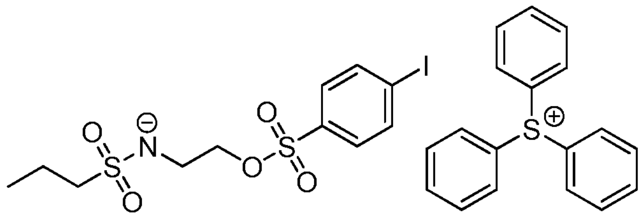 |
| Salt compound 11 | 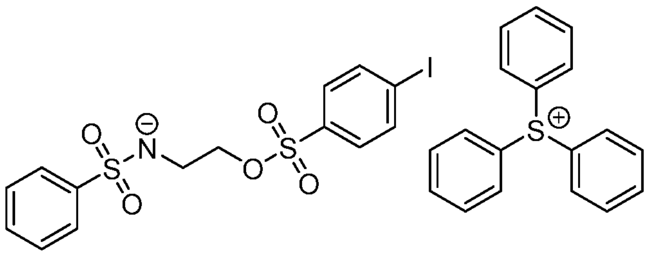 |
| Salt compound 12 | 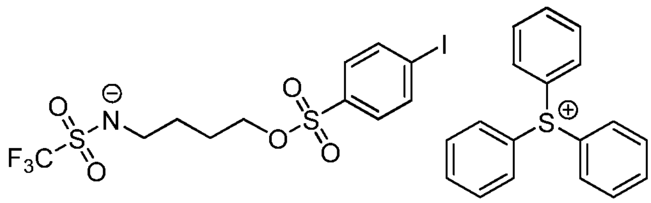 |

**Example 1-1: Salt Compound 1**

**Step 1**) Preparation of 1,1,1-trifluoro-N-(2-hydroxyethyl)methanesulfoneamide

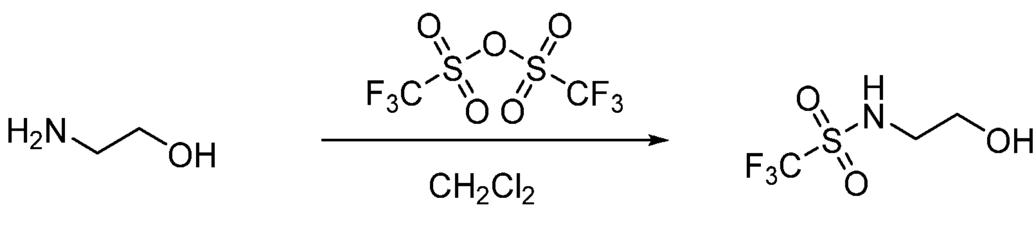


Ethanolamine(20g) was dissolved in dichloromethane(150g) to prepare a mixture 1, and this mixture 1 was cooled to -78°C and stirred. Then, trifluoromethanesulfonic anhydride(97g) and dichloromethane(150g) were mixed to prepare a mixture 2, and the mixture 2 was slowly added dropwise to the mixture 1 over about 1 hour. After completing the dropwise addition, the temperature was further maintained at -78°C for 1 hour, and then gradually raised to 0°C, and the mixture was further stirred for 5 hours. The reaction was terminated with distilled water, the organic layer was washed three times with distilled water to remove unreacted materials, and then concentrated under reduced pressure and then subjected to column chromatography to give 1,1,1-trifluoro-N-(2-hydroxyethyl)methanesulfonamide (35.7g, yield: 56.4%).

$^1$H-NMR($CDCl_3$, TMS(standard material), 600MHz): 8.2(NH, s, 1H), 4.5($CH_2$, t, 2H), 3.7($CH_2$, t, 2H), 1.3(OH, s, 1H)

**Step 2**) Preparation of 2-(trifluoromethylsulfoneamido)ethyl 4-iodobenzenesulfonate

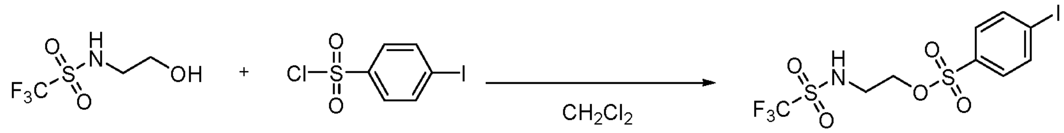


1,1,1-Trifluoro-N-(2-hydroxyethyl)methanesulfonamide(30 g) obtained in step 1) was dissolved in dichloromethane(200 g), and a mixture of 4-iodobenzene-1-sulfonylchloride(50 g) and dichloromethane(200 g) was slowly added dropwise while stirring, and allowed to react for 3 hours. Next, the organic layer was washed with distilled water until it became neutral (about pH 6-7), and the organic layer was concentrated under reduced pressure to give 2-(trifluoromethylsulfonamido)ethyl 4-iodobenzenesulfonate(62.3g, yield: 87.3%).

$^1$H-NMR($CDCl_3$, TMS, 600MHz): 8.5(NH, s, 1H), 8.2(CH, d, 2H), 7.5(CH, d, 2H), 4.5($CH_2$, t, 2H), 3.9($CH_2$, t, 2H)

**Step 3**) Preparation of triphenylsulfonium (2-(4-iodophenylsulfonyloxy)ethyl)(trifluoromethanesulfonyl)amide) (salt compound 1)

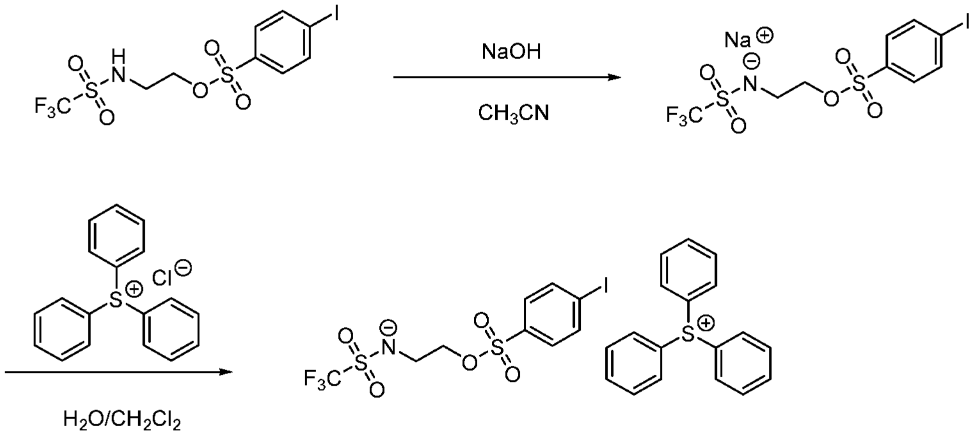


2-(Trifluoromethylsulfonamido)ethyl 4-iodobenzenesulfonate (30 g) obtained from step 2) was dissolved in acetonitrile(200g), 10% sodium hydroxide solution(28g) was slowly added thereto, and the mixture was stirred at room temperature for 3 hours and then distilled under reduced pressure to give sodium (2-(4-iodophenylsulfonyloxy)ethyl)(trifluoromethanesulfonyl)amide salt, about 30.1g, yield: 98.54%). Next, triphenylsulfoniumchloride(20.56g), distilled water(200g), dichloromethane (300g) and tetrabutylammonium fluoride (0.5g) as a catalyst were added thereto, and sufficiently stirred at room temperature for about 12 hours. The organic layer was separated, washed with distilled water until it became neutral (about pH 6-7), and then concentrated under reduced pressure. The process of adding ethyl acetate(100g) to the resulting concentrate, stirring the mixture and removing the organic solvent was repeated three times, and the product was concentrated under reduced pressure to give triphenylsulfonium (2-(4-iodophenylsulfonyloxy) ethyl)(trifluoromethanesulfonyl)amide, salt compound 1) (39g, yield: 87.2%).

$^1$H-NMR($CDCl_3$, TMS, 600MHz): 8.24(CH, d, 2H), 7.54(CH, d, 2H), 7.27-7.78(CH, m, 15H), 4.5($CH_2$, t, 2H), 3.7($CH_2$, t, 2H)

**Example 1-2: Salt Compound 2**

Triphenylsulfonium (2-(4-iodophenylsulfonyloxy)ethyl)(trifluoromethanesulfonyl)amide (salt compound 2) was prepared in the same manner as in Example 1-1, through an ion exchange reaction using adamantanesulfonylchloride instead of 4-iodobenzene-1-sulfonylchloride in step 2).

**Example 1-3: Salt Compound 3**

Triphenylsulfonium (2-(4-methoxyphenylsulfonyloxy)ethyl)(trifluoromethanesulfonyl)amide (salt compound 3) was prepared in the same manner as in Example 1-1, through an ion exchange reaction using 4-methoxybenzenesulfonyl chloride instead of 4-iodobenzene-1-sulfonylchloride in step 2).

**Example 1-4: Salt Compound 4**

Triphenylsulfonium (2-(4-methoxyphenylsulfonyloxy)ethyl)(trifluoromethanesulfonyl)amide (salt compound 4) was prepared in the same manner as in Example 1-1, through an ion exchange reaction using benzo-1,4-dioxanesulfonyl chloride instead of 4-iodobenzene-1-sulfonylchloride in step 2),

**Example 1-5: Salt Compound 5**

Triphenylsulfonium (2-(4-iodophenylsulfonyloxy)ethyl)(trifluorobenzenesulfonyl)amide (salt compound 5) was prepared in the same manner as in Example 1-1, through an ion exchange reaction using 2,3,4,5,6-pentafluoro-N-(2-hydroxyethyl)benzenesulfoneamide instead of 1,1,1-trifluoro-N-(2-hydroxyethyl)methanesulfonamide in step 2).

**Example 1-6: Salt Compound 6**

Triphenylsulfonium (2-(naphthalenesulfonyloxy)ethyl)(trifluoromethanesulfonyl)amide (salt compound 6) was prepared in the same manner as in Example 1-1, through an ion exchange reaction using naphthalene-sulfonyl chloride instead of 4-iodobenzene-1-sulfonyl chloride in step 2).

**Example 1-7: Salt Compound 7**

Diphenyl (4-(4-iodobenzene-1-sulfonyloxy)phenyl)sulfonium (2-(naphthalenesulfonyloxy)ethyl)(trifluoromethanesulfonyl)amide (salt compound 7) was prepared in the same manner as in Example 1-1, through an ion exchange reaction using diphenyl (4-(1-iodobenzene-4-sulfonyloxy)phenyl)sulfonium chloride instead of triphenylsulfonium chloride in step 3).

**Example 1-8: Salt Compound 8**

Triphenylsulfonium (2-(propanesulfonyloxy)ethyl)(trifluoromethanesulfonyl)amide (salt compound 8) was prepared in the same manner as in Example 1-1, through an ion exchange reaction using propanesulfonyl chloride instead of 4-iodobenzene-1-sulfonylchloride in step 2).

**Example 1-9: Salt Compound 9**

Triphenylsulfonium (2-(1,4-dioxan-2-yl)ethanesulfonyloxy)ethyl)(trifluoromethanesulfonyl)amide (salt compound 9) was prepared in the same manner as in Example 1-1, through an ion exchange reaction using 2-(1,4-dioxan-2-yl)ethanesulfonyl chloride instead of 4-iodobenzene-1-sulfonylchloride in step 2).

**Example 1-10: Salt Compound 10**

Triphenylsulfonium (2-(4-iodophenylsulfonyloxy)ethyl)(propanesulfonyl)amide (salt compound 10) was prepared in the same manner as in Example 1-1, through an ion exchange reaction using N-(2-hydroxyethyl)propanesulfoneamide instead of trifluoro-N-(2-hydroxyethyl)methanesulfonamide in step 2).

**Example 1-11: Salt Compound 11**

Triphenylsulfonium (2-(4-iodophenylsulfonyloxy)ethyl)(benzenesulfonyl)amide (salt compound 11) was prepared in the same manner as in Example 1-1, through an ion exchange reaction using N-(2-hydroxyethyl)benzenesulfoneamide instead of trifluoro-N-(2-hydroxyethyl)methanesulfonamide in step 2).

**Example 1-12: Salt Compound 12**

Triphenylsulfonium (2-(4-iodophenylsulfonyloxy)butyl)(trifluoromethanesulfone)amide (salt compound 12) was

prepared in the same manner as in Example 1-1, through an ion exchange reaction using 1,1,1-trifluoro-N-(2-hydroxybutyl)methanesulfoneamide instead of trifluoro-N-(2-hydroxyethyl)methanesulfonamide in step 2.

**Comparative Example 1-1: Salt Compound 13**

Salt compound 13 having the following structure was used.

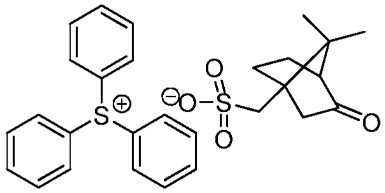

**Comparative Example 1-2: Salt Compound 14**

Salt compound 14 having the following structure was used.

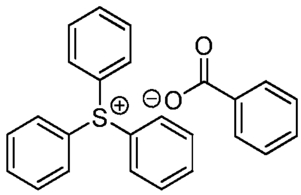

**Comparative Example 1-3: Salt Compound 15**

Salt compound 15 having the following structure was used.

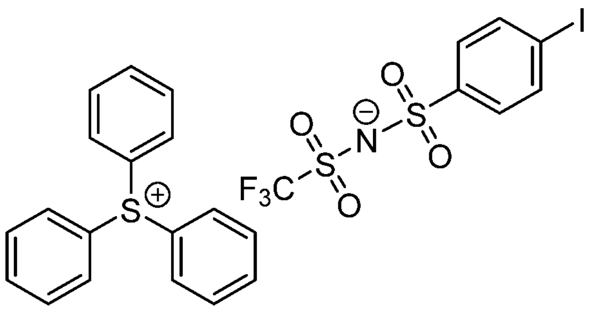

**<Synthesis Example: Preparation of base polymer>**

**Synthesis Example 1: Preparation of Polymer 1**

In accordance with the repeating units shown in Table 2 below, monomers were combined and dissolved in tetrahydrofuran (THF) to prepare a reaction solution, which was subjected to a copolymerization reaction. Then, for low molecule removal and solidification, the reaction solution was added dropwise to methanol, n-hexane or hexane/isopropyl alcohol, or methanol/water mixed solvent, washing was repeated with the same solvent, and then separated and dried to prepare a base polymer (referred to as polymer 1). For monomers containing a hydroxy styrene group, this was used in a state protected with an acetoxy group, and after the primary polymerization, a secondary hydrolysis reaction was further advanced, and a step of precipitation with water was added.

**Synthesis Examples 2 to 4: Preparation of polymers 2 to 4**

Polymers 2 to 4 were prepared in the same manner as in Synthesis Example 1, except that the type and amount of monomers were changed in accordance with the repeating units shown in Table 2 below.

After drying the base polymer prepared in Synthesis Examples 1 to 4, the constituent components of the

monomer were analyzed by $^{1}H$ or $^{13}C$-NMR spectroscopy, Mw and PDI (polydispersity index, expressed as Mw/Mn) were analyzed using gel permeation chromatography (GPC), and the polymer molecular weight (Mw) expressed in terms of standard polystyrene was determined using THF solvent. The types and analysis results of repeating units of polymers 1 to 4 are listed in Table 2 below.

[Table 2] Type, structure, molecular weight(Mw), and PDI(Mw/Mn) of repeating units of polymers 1 to 4 prepared in Synthesis Examples 1 to 4

| Type | Structure, molecular weight, and PDI values of repeating units |
|---|---|
| Polymer 1 | 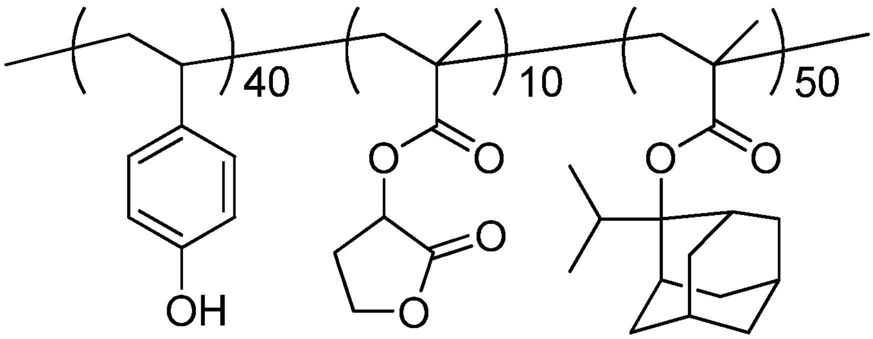 Mw=6,900, PDI(Mw/Mn)=1.46 |
| Polymer 2 | 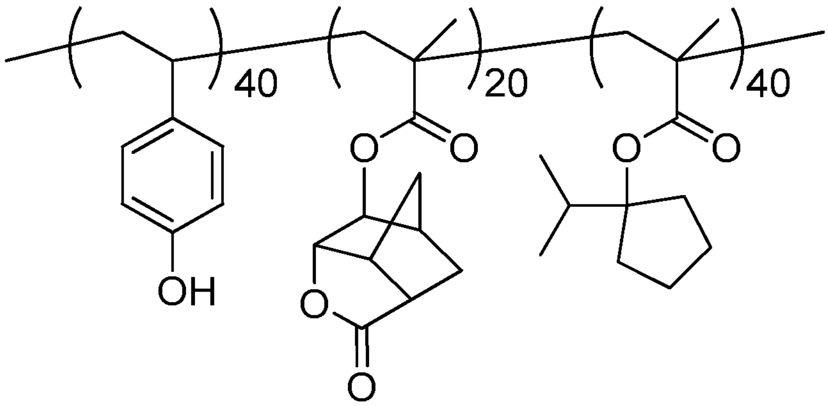 Mw=6,500, PDI(Mw/Mn)=1.43 |
| Polymer 3 | 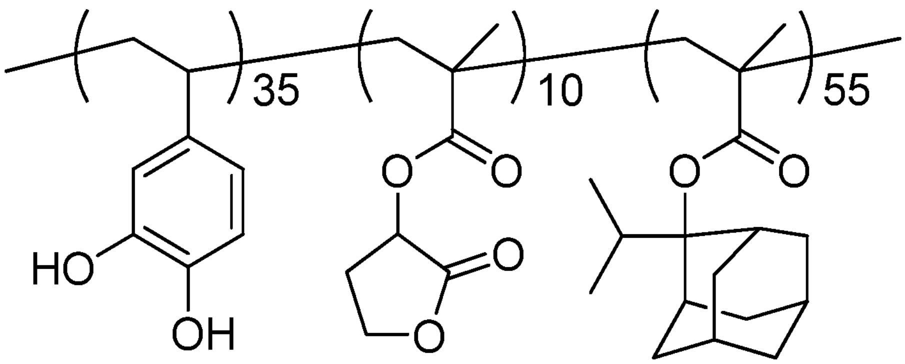 Mw=6,600, PDI(Mw/Mn)=1.64 |

(continued)

| Type | Structure, molecular weight, and PDI values of repeating units |
|---|---|
| Polymer 4 | 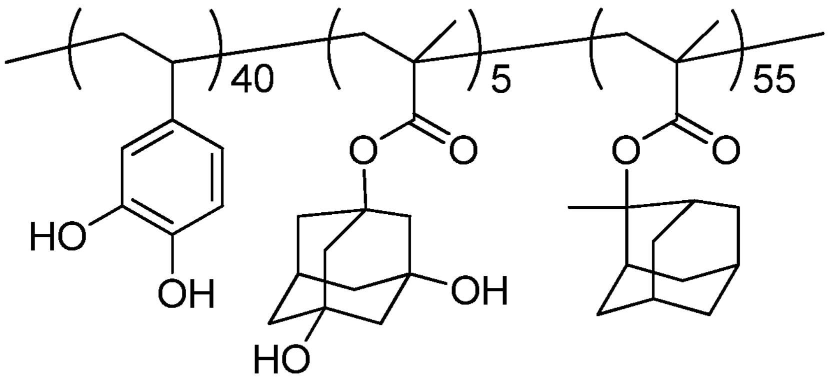 Mw=7,100, PDI(Mw/Mn)=1.67 |

**<Example 2: Preparation of photoresist composition>**

In accordance with the components listed in Table 3 below, the base polymer, the photoacid generator, and the quencher were dissolved in an organic solvent, and filtered through a polytetrafluoroethylene (PTFE) filter having pores of 0.2 μm size to prepare the photoresist compositions of Examples 2-1 to 2-25 and Comparative Examples 2-1 to 2-6.

**<<Base polymer>>**

In Examples 2-1 to 2-25 and Comparative Examples 2-1 to 2-6, one of the polymers 1 to 4 prepared in Synthesis Examples 1 to 4 was selected and used as a base polymer.

**<<Photoacid generator>>**

In Examples 2-1 to 2-25 and Comparative Examples 2-1 to 2-6, one of the compounds having the following structure was selected and used as a photoacid generator (PAG).

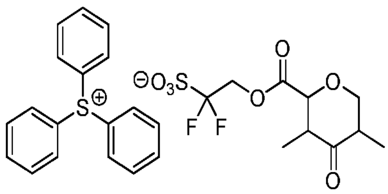

PAG-1

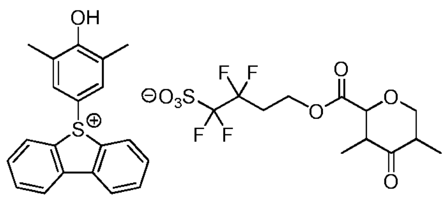

PAG-2

**<<Quencher>>**

In Examples 2-1 to 2-25, the salt compounds 1 to 12 prepared in Examples 1-1 to 1-12 were used as quenchers. In Comparative Examples 2-1 to 2-6, the salt compounds 13 to 15 prepared in Comparative Examples 1-1 to 1-3 were used as quenchers.

<<Organic Solvent>>

In Examples 2-1 to 2-25 and Comparative Examples 2-1 to 2-6, one or more of PGMEA (propylene glycol monomethyl ether acetate), PGME (propylene glycol monomethyl ether), EL (ethyl lactate), and CyH (cyclohexanone) were selected and used as an organic solvent.

[Table 3] Components and contents of the photoresist compositions of Examples 2-1 to 2-25 and Comparative Examples 2-1 to 2-6

| Example | | | | |
|---|---|---|---|---|
| Example No. | Base polymer (part by weight) | Photoacid generator (part by weight) | Quencher (part by weight) | Organic solvent (part by weight) |
| 2-1 | Polymer 1 (100) | PAG 1 (15) | Salt compound 1 (18) | PGMEA(1300) PGME(1300) |
| 2-2 | Polymer 1 (100) | PAG 1 (15) | Salt compound 2 (18.5) | PGMEA(1300) PGME(1300) |
| 2-3 | Polymer 1 (100) | PAG 1 (15) | Salt compound 3 (18) | PGMEA(1300) PGME(1300) |
| 2-4 | Polymer 1 (100) | PAG 1 (15) | Salt compound 4 (19) | PGMEA(1300) PGME(1300) |
| 2-5 | Polymer 1 (100) | PAG 1 (15) | Salt compound 5 (18) | PGMEA(1300) PGME(1300) |
| 2-6 | Polymer 1 (100) | PAG 1 (15) | Salt compound 6 (19) | PGMEA(1300) PGME(1300) |
| 2-7 | Polymer 1 (100) | PAG 1 (15) | Salt compound 7 (21) | PGMEA(1300) PGME(1300) |
| 2-8 | Polymer 1 (100) | PAG 1 (15) | Salt compound 8 (18) | PGMEA(1300) PGME(1300) |
| 2-9 | Polymer 1 (100) | PAG 1 (15) | Salt compound 9 (19) | PGMEA(1300) PGME(1300) |
| 2-10 | Polymer 1 (100) | PAG 1 (15) | Salt compound 10 (18) | PGMEA(1300) PGME(1300) |
| 2-11 | Polymer 1 (100) | PAG 1 (15) | Salt compound 11 (20) | PGMEA(1300) PGME(1300) |
| 2-12 | Polymer 1 (100) | PAG 1 (15) | Salt compound 12 (20.5) | PGMEA(1300) PGME(1300) |
| 2-13 | Polymer 1 (100) | PAG 2 (17) | Salt compound 1 (18) | PGMEA(1300) PGME(1300) |
| 2-14 | Polymer 1 (100) | PAG 2 (17) | Salt compound 2 (18.5) | PGMEA(1300) PGME(1300) |
| 2-15 | Polymer 2 (100) | PAG 1 (15) | Salt compound 1 (18) | PGMEA(1200) PGME(1400) |
| 2-16 | Polymer 2 (100) | PAG 2 (17) | Salt compound 1 (20) | PGMEA(1200) PGME(1400) |
| 2-17 | Polymer 2 (100) | PAG 2 (17) | Salt compound 5 (22) | PGMEA(1200) PGME(1400) |
| 2-18 | Polymer 2 (100) | PAG 2 (17) | Salt compound 7 (23) | PGMEA(1200) PGME(1400) |
| 2-19 | Polymer 3 (100) | PAG 1 (15) | Salt compound 1 (19.5) | PGMEA(1600) EL(1000) |

(continued)

| Example | | | | |
|---|---|---|---|---|
| Example No. | Base polymer (part by weight) | Photoacid generator (part by weight) | Quencher (part by weight) | Organic solvent (part by weight) |
| 2-20 | Polymer 3 (100) | PAG 2 (17) | Salt compound 2 (19) | PGMEA(1600) EL(1000) |
| 2-21 | Polymer 3 (100) | PAG 2 (17) | Salt compound 3 (20) | PGMEA(1600) EL(1000) |
| 2-22 | Polymer 3 (100) | PAG 2 (17) | Salt compound 7 (22) | PGMEA(1600) EL(1000) |
| 2-23 | Polymer 4 (100) | PAG 1 (15) | Salt compound 1 (21) | PGMEA(1600) CyH(1000) |
| 2-24 | Polymer 4 (100) | PAG 2 (17) | Salt compound 2 (22.5) | PGMEA(1600) CyH(1000) |
| 2-25 | Polymer 4 (100) | PAG 2 (17) | Salt compound 5 (20.5) | PGMEA(1600) CyH(1000) |
| Comparative Example | | | | |
| Comparative Example No. | Base polymer (part by weight) | Photoacid generator (part by weight) | Quencher (part by weight) | Solvent (part by weight) |
| 2-1 | Polymer 1 (100) | PAG 1 (25) | Salt compound 13 (12) | PGMEA(1300) PGME(1300) |
| 2-2 | Polymer 1 (100) | PAG 2 (25) | Salt compound 14 (16) | PGMEA(1300) PGME(1300) |
| 2-3 | Polymer 2 (100) | PAG 2 (25) | Salt compound 14 (18) | PGMEA(1200) PGME(1400) |
| 2-4 | Polymer 3 (100) | PAG 2 (25) | Salt compound 14 (18) | PGMEA(1600) EL(1000) |
| 2-5 | Polymer 4 (100) | PAG 2 (25) | Salt compound 14 (18) | PGMEA(1600) CyH(1000) |
| 2-6 | Polymer 1 (100) | PAG 1 (15) | Salt compound 15 (18) | PGMEA(1300) PGME(1300) |

**<Experimental Example>**

**Experimental Example 1: Resist pattern formation experiment**

To analyze resist pattern characteristics, an organic thin film under the resist was applied onto a silicon wafer to prepare a substrate, and the photoresist compositions of Examples 2-1 to 2-20 and Comparative Examples 2-1 to 2-6 were spin coated onto the substrate to form a resist thin film. Next, the resist thin film was heated (prebaked) at a temperature of 100°C to 130°C for 60 seconds using a hot plate, (i) exposed with ASML NXE:3400 EUV exposure equipment with a numerical aperture (N.A.) of 0.33, and then heated (post exposure bake, PEB) at a temperature of 100°C to 130°C for 60 seconds. The thus heated wafer was developed with a 2.38 wt.% tetramethylammonium hydroxide (TMAH) aqueous solution for 30 seconds to form a 1:1 line and space (L/S: line/space) pattern with a film thickness of 40 nm and a line width of 20 nm.

### Experimental Example 2: Evaluation of resist pattern characteristics

To measure the pattern size of the patterned wafer of Experimental Example 1, the pattern size was measured using a Critical Dimension Scanning Electron Microscope (CD-SEM, product name: CG-5000) from Hitachi High-Technologies Corp., and the sensitivity, line edge roughness(LER), and process margins(EL and DOF) were evaluated as follows.

**(1) Sensitivity evaluation**

The optimal exposure energy, Eop (mJ/cm$^2$) for forming a resist pattern having the same size space (pitch 40 nm) with respect to a resist line width of 20 nm was measured. The lower the LER and the larger the process margin (EL, DOF) while having a lower sensitivity value can be evaluated as superior.

**(2) LER evaluation** (Line Edge Roughness)

In the 20nm pattern formed at the optimal exposure energy (Eop), the average value of three times(3s) the standard deviation(s) for the value of line edge roughness measured at 200 measurement points in the length direction of line width pattern was calculated. As the LER value is lower, it can be evaluated as superior.

**(3) EL margin evaluation** (Exposure Latitude, unit: %)

It was calculated according to the following Calculation Equation using the exposure energy value when a 20 nm sized pattern formed at the optimal exposure energy (Eop) was formed within the range of 20 nm ± 5% line width (19 nm to 21 nm). As the EL margin value is higher, it can be evaluated as superior.

$$\text{EL margin (\%)} = (|E_1 - E_2|/\text{Eop}) \times 100$$

$E_1$: exposure energy value (mJ/cm$^2$) when forming a pattern with a line width of 19nm
$E_2$: exposure energy value (mJ/cm$^2$) when forming a pattern with a line width of 21 nm

**(4) DOF margin evaluation** (Depth of focus, unit: nm)

It was calculated by a depth of focus change value having a value within the range of ±1.5% (19.7nm to 20.3nm) of the 20nm pattern line width of 20nm according to the change(±) in depth of focus (DOF) from the optimal exposure energy(Eop). The Equation for this is as follows. As the DOF margin value is higher, it can be evaluated as superior.

$$\text{DOF margin (nm)} = D_1 + D_2$$

wherein, $D_1$: +depth of focus value (nm) when a pattern within ±1.5% of the line width is formed.
$D_2$: -depth of focus value (nm) when a pattern within ±1.5% of the line width is formed

The evaluation results of the above-mentioned resist pattern characteristics are listed in Table 4 below.

[Table 4] Evaluation results of resist characteristics using the photoresist compositions of Examples 2-1 to 2-25 and Comparative Examples 2-1 to 2-6

| Example | | | | | |
|---|---|---|---|---|---|
| Example No. | PEB (Post exposure bake: heating temperature after exposure) (°C) | Sensitivity (mJ/cm$^2$) | LER (nm) | EL (%) | DOF (nm) |
| 2-1 | 100 | 62 | 2.42 | 11.2 | 100 |
| 2-2 | 100 | 66 | 2.43 | 11.9 | 100 |
| 2-3 | 100 | 63 | 2.61 | 11.8 | 90 |
| 2-4 | 100 | 65 | 2.54 | 11.2 | 90 |

(continued)

| Example | | | | | |
|---|---|---|---|---|---|
| Example No. | PEB (Post exposure bake: heating temperature after exposure) (°C) | Sensitivity ($mJ/cm^2$) | LER (nm) | EL (%) | DOF (nm) |
| 2-5 | 100 | 68 | 2.56 | 12.1 | 100 |
| 2-6 | 100 | 65 | 2.61 | 11.6 | 90 |
| 2-7 | 100 | 64 | 2.48 | 107 | 100 |
| 2-8 | 100 | 72 | 2.67 | 9.8 | 80 |
| 2-9 | 100 | 74 | 2.61 | 10.9 | 90 |
| 2-10 | 100 | 70 | 2.81 | 9.1 | 80 |
| 2-11 | 100 | 68 | 2.63 | 10.2 | 80 |
| 2-12 | 100 | 69 | 284 | 108 | 80 |
| 2-13 | 100 | 60 | 2.37 | 106 | 90 |
| 2-14 | 100 | 64 | 2.41 | 107 | 90 |
| 2-15 | 100 | 66 | 2.38 | 12.4 | 80 |
| 2-16 | 100 | 63 | 234 | 11.6 | 90 |
| 2-17 | 100 | 67 | 264 | 12.2 | 100 |
| 2-18 | 100 | 62 | 2.39 | 12.3 | 90 |
| 2-19 | 100 | 61 | 2.54 | 106 | 90 |
| 2-20 | 100 | 58 | 2.47 | 11.1 | 80 |
| 2-21 | 100 | 57 | 2.55 | 11.3 | 100 |
| 2-22 | 100 | 55 | 2.38 | 10.9 | 90 |
| 2-23 | 115 | 68 | 2.66 | 12.4 | 80 |
| 2-24 | 115 | 64 | 2.64 | 11.8 | 90 |
| 2-25 | 115 | 66 | 274 | 122 | 80 |
| Comparative Example | | | | | |
| Comparative Example No. | PEB (°C) | Sensitivity ($mJ/cm^2$) | LER (nm) | EL (%) | DOF (nm) |
| 2-1 | 100 | 82 | 3.58 | 9.7 | 70 |
| 2-2 | 100 | 84 | 3.25 | 9.3 | 80 |
| 2-3 | 100 | 83 | 3.26 | 10.8 | 80 |
| 2-4 | 100 | 79 | 3.33 | 10.2 | 80 |
| 2-5 | 115 | 86 | 3.27 | 11.9 | 80 |
| 2-6 | 100 | 64 | 3.61 | 8.4 | 60 |

Referring to Table 4, when the photoresist compositions according to Examples 2-1 to 2-25 were used, it showed a sensitivity of 55 $mJ/cm^2$ or more and 74 $mJ/cm^2$ or less, a LER of 2.34 nm or more and 2.84 nm or less, an EL margin of 9.1% or more and 12.4% or less, and a DOF margin of 80 nm or more and 100 nm or less.

On the other hand, when the photoresist compositions of Comparative Examples 2-1 to 2-5 were used, it showed a sensitivity of 79 $mJ/cm^2$ or more and 86 $mJ/cm^2$ or less, a LER of 3.25 nm or more and 3.58 nm or less, an EL margin of 9.3% or more and 11.9% or less, and a DOF margin of 70 nm or more and 80 nm or less, confirming that they had poor physical properties as compared to Examples. In particular, Comparative Example 2-6 using the salt compound 14 had excellent sensitivity, but showed a LER of 3.61 nm and a DOF margin of 60 nm, confirming that it had poorer physical properties compared to Examples.

## Claims

1. A salt compound comprising an anion represented by the following Chemical Formula 1, and a cation represented by the following Chemical Formula 2:

[Chemical Formula 1]

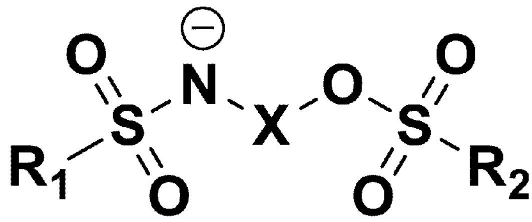

[Chemical Formula 2]

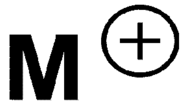

wherein, in Chemical Formulas 1 and 2,
$R_1$ and $R_2$ are the same as or different from each other, and are each independently one of an alkyl group, an alkenyl group, a cycloalkyl group, a cycloalkenyl group, an aryl group, a halogen, or a combination thereof,
X is one of a direct bond, an alkylene group, an alkenylene group, a cycloalkylene group, a cycloalkenylene group, an arylene group, or a combination thereof, and
$M^+$ is either an onium ion or a metal ion.

2. The salt compound according to claim 1, wherein: the X is an alkylene group or alkenylene group.

3. The salt compound according to claim 1, wherein: the X is an alkylene group having 2 to 3 carbon atoms.

4. The salt compound according to claim 1, wherein: the $R_1$ is one of an alkyl group having 1 to 30 carbon atoms in which at least one hydrogen atom is substituted with halogen, an aryl group having 6 to 30 carbon atoms in which at least one hydrogen atom is substituted with halogen, or an aryl group having 6 to 30 carbon atoms in which at least one hydrogen atom is substituted with a halogenated alkyl group.

5. The salt compound according to claim 1, wherein: the $R_1$ is one of an alkyl group having 1 to 30 carbon atoms in which 2 or more and 6 or less hydrogen atoms are substituted with halogen, or an aryl group having 6 to 30 carbon atoms in which 2 or more and 6 or less hydrogen atoms are substituted with halogen.

6. The salt compound according to claim 1, wherein: the $R_2$ is a substituted or unsubstituted aryl group.

7. The salt compound according to claim 1, wherein: the $R_2$ is one of an aryl group in which at least one hydrogen atom is substituted with halogen, an aryl group in which a hydrogen atom is unsubstituted, or an aryl group in which at least one hydrogen atom is substituted with a heteroalkyl group.

8. The salt compound according to claim 1, wherein: the $R_2$ is an aryl group in which at least one hydrogen atom is substituted with halogen.

9. The salt compound according to claim 1, wherein:

the $R_1$ is one of methyl, ethyl, propyl, halogen, trifluoromethyl, cyclohexyl, adamantyl, phenyl, 4-fluorophenyl, 4-bromophenyl, 4-iodophenyl, 2,4-difluorophenyl, 2,5-difluorophenyl, 4-(trifluoromethyl)phenyl, or 1,2,3,4,5-pentafluorophenyl.

**10.** The salt compound according to claim 1, wherein:
the $R_2$ is one of methyl, ethyl, propyl, trifluoromethyl, cyclohexyl, adamantyl, acetyl-4-piperidinyl, tert-butoxycarbonyl-4-piperidinyl, 4-fluorophenyl, 4-bromophenyl, 4-iodophenyl, 2,5-difluorophenyl, 2,4-difluorophenyl, 1,2,3,4,5-pentafluorophenyl, 4-methoxyphenyl, 1-naphthyl, 2-naphthyl, 4-pyridinyl, 6-benzooxazolyl, 5-phthalanyl, 1,4-dioxan-2-ylethyl, or 1,4-benzodioxan-6-yl.

**11.** The salt compound according to claim 1, wherein:
the $M^+$ is one of a sulfonium ion, an iodonium ion, or an ammonium ion.

**12.** The salt compound according to claim 1, wherein:
the anion represented by Chemical Formula 1 is any one selected from the group consisting of:

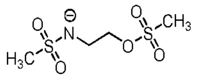
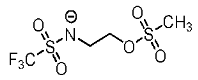
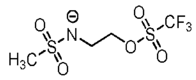
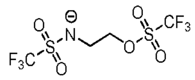
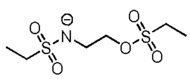
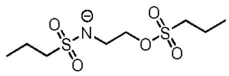
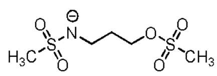
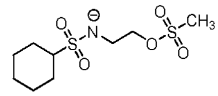
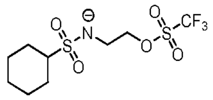
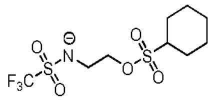
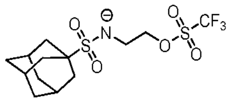
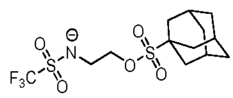
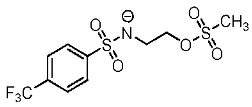
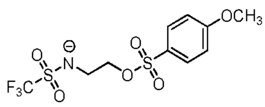
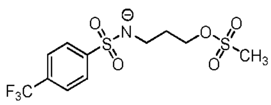
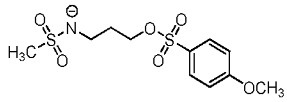
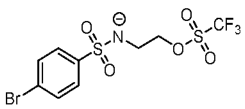
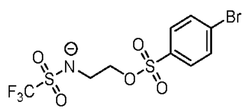
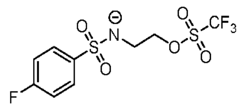
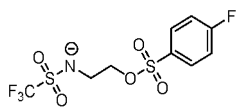
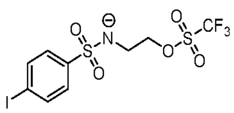
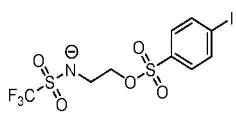
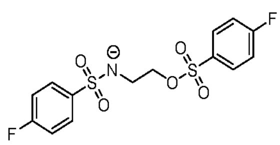
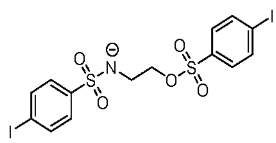
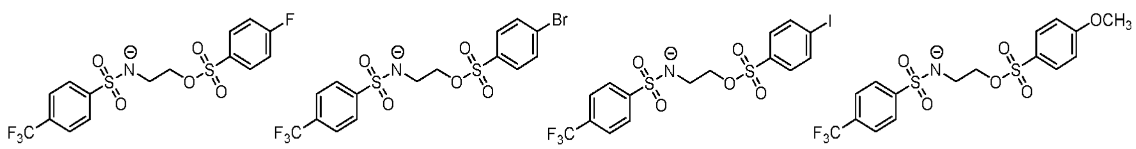
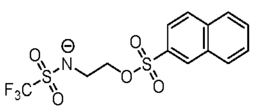
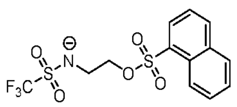
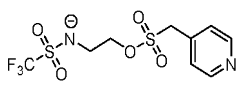
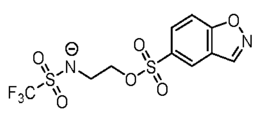

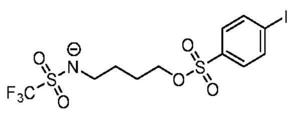 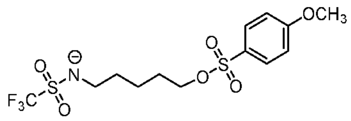 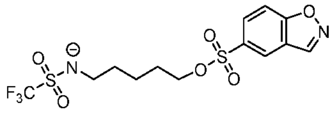

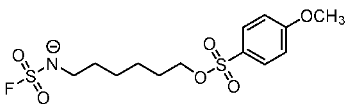 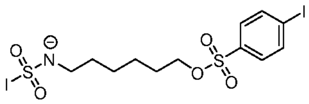 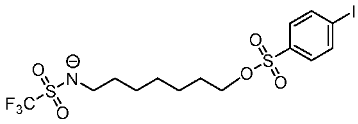

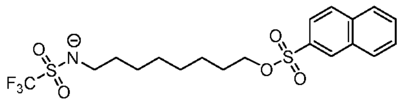

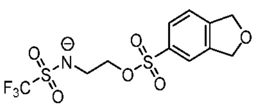 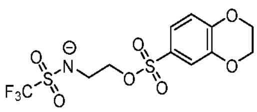 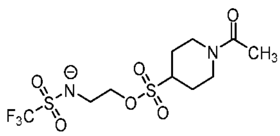 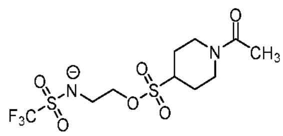

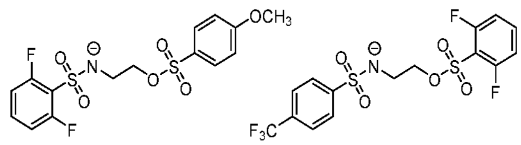 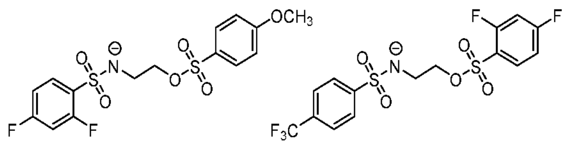

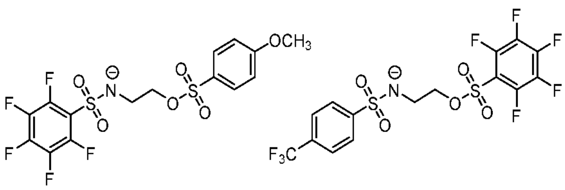 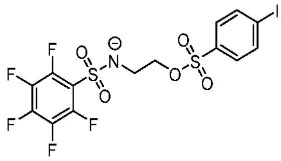 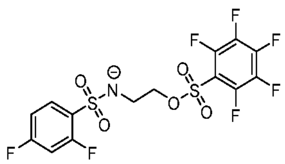

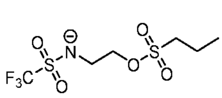 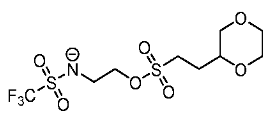 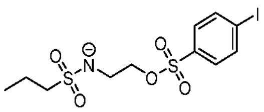 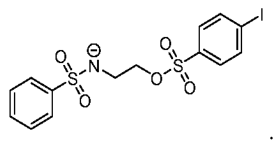.

**13.** The salt compound according to claim 1, wherein:
the cation represented by Chemical Formula 2 is any one selected from the group consisting of:

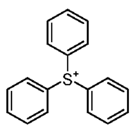 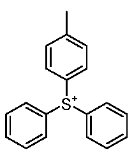 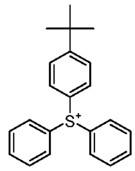 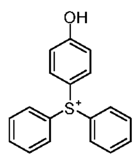

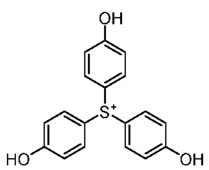 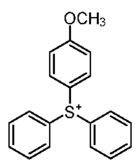 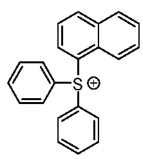 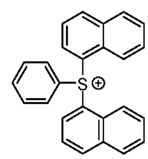

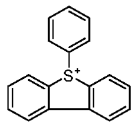 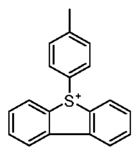 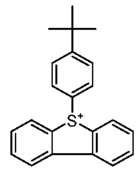 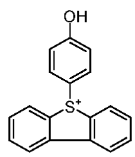

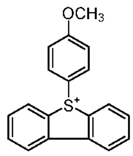 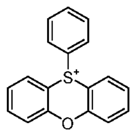 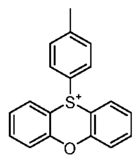 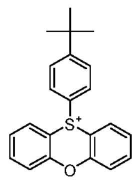

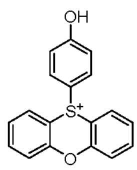 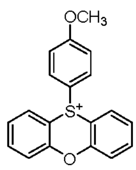 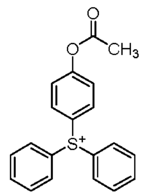 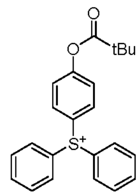

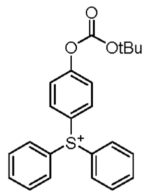 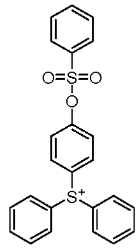 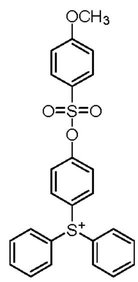 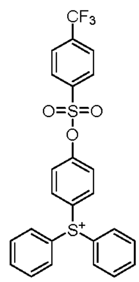

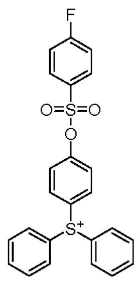 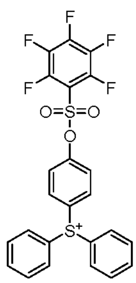 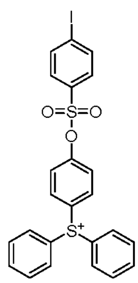 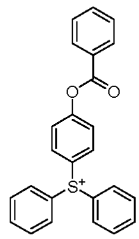

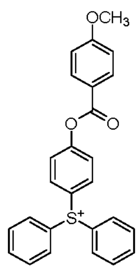 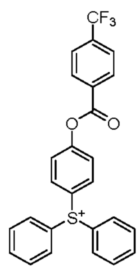 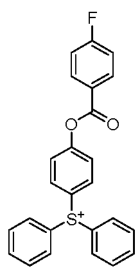 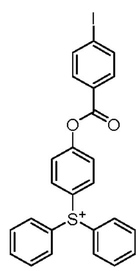

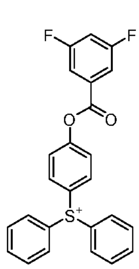 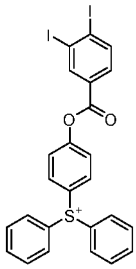 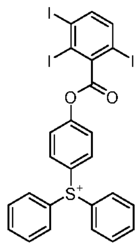 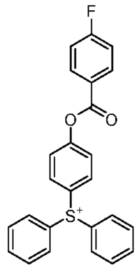

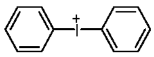 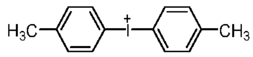 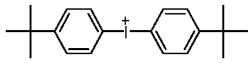 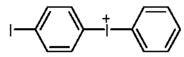.

**14.** A quencher comprising the salt compound according to claim 1.

**15.** A photoresist composition comprising:

the salt compound according to claim 1;
a photoacid generator;
a base polymer; and
an organic solvent.

## INTERNATIONAL SEARCH REPORT

International application No.
**PCT/KR2021/019567**

### A. CLASSIFICATION OF SUBJECT MATTER

**C07C 311/06**(2006.01)i; **C07C 311/14**(2006.01)i; **C07C 311/17**(2006.01)i; **C07C 321/28**(2006.01)i; **C07C 323/22**(2006.01)i; **C07C 323/66**(2006.01)i; **C07C 25/18**(2006.01)i; **C07D 319/20**(2006.01)i; **G03F 7/004**(2006.01)i

According to International Patent Classification (IPC) or to both national classification and IPC

### B. FIELDS SEARCHED

Minimum documentation searched (classification system followed by classification symbols)

C07C 311/06(2006.01); C07D 285/04(2006.01); G03F 7/004(2006.01); G03F 7/039(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 염(salt), 술폰네이트(sulfonate), 술포닐아미노(sulfonylamino), 산확산 억제제(acid diffusion inhibitor), 포토레지스트(photoresist)

### C. DOCUMENTS CONSIDERED TO BE RELEVANT

| Category* | Citation of document, with indication, where appropriate, of the relevant passages | Relevant to claim No. |
|---|---|---|
| X<br>A | KITA, F. et al. On the characteristics of electrolytes with new lithium imide salts. Journal of power sources . 1997, vol. 68, no. 2, pp. 307-310.<br>See abstract; and table 1. | 1,4,10<br>2,3,5-9,11-15 |
| X | Chemical abstract. 1986, DN(Document Number) 105:32094. [BRINK, K. et al. IR and Raman spectra of methylsulfonylhydroxylamines. Spectrochimica acta, part A: molecular and biomolecular spectroscopy. 19 86, vol. 42A, no. 4, pp. 525-529].<br>See abstract; and RN 103088-26-2. | 1,9,10 |
| A | JP 2018-060069 A (SHIN ETSU CHEM. CO., LTD.) 12 April 2018 (2018-04-12)<br>See abstract; claims 1-15; and paragraphs [0031]-[0040]. | 1-15 |
| A | KR 10-2018-0111596 A (TOKYO OHKA KOGYO CO., LTD.) 11 October 2018 (2018-10-11)<br>See claims 1-8. | 1-15 |
| PX | WO 2021-251086 A1 (FUJIFILM CORPORATION) 16 December 2021.<br>See paragraphs [0311] and [0313]; and compound X-8. | 1,4,5,11,13 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **05 September 2022** | **05 September 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2021/019567**

| Patent document cited in search report | Publication date (day/month/year) | Patent family member(s) | Publication date (day/month/year) |
|---|---|---|---|
| JP 2018-060069 A | 12 April 2018 | CN 107918248 A | 17 April 2018 |
| | | CN 107918248 B | 10 September 2021 |
| | | JP 6980993 B2 | 15 December 2021 |
| | | KR 10-1986425 B1 | 05 June 2019 |
| | | KR 10-2018-0038394 A | 16 April 2018 |
| | | TW 201823333 A | 01 July 2018 |
| | | TW I657106 B | 21 April 2019 |
| | | US 10474030 B2 | 12 November 2019 |
| | | US 2018-0101094 A1 | 12 April 2018 |
| KR 10-2018-0111596 A | 11 October 2018 | CN 108693711 A | 23 October 2018 |
| | | JP 2018-173606 A | 08 November 2018 |
| | | JP 6890454 B2 | 18 June 2021 |
| | | TW 201902883 A | 16 January 2019 |
| | | US 10514600 B2 | 24 December 2019 |
| | | US 2018-0284610 A1 | 04 October 2018 |

Form PCT/ISA/210 (patent family annex) (July 2019)